# EUROPEAN PATENT APPLICATION

(11) **EP 4 825 955 A1**
(43) Date of publication of application: **21.10.2026**
(21) Application number: 25171233.7
(22) Date of filing: 17.04.2025
(51) Int. Cl.: C12M 1/00, C12M 3/00

(54) **DEVICE FOR SEPARATING MOTILE CELLS**

(71) Applicant: Minitüb GmbH, 84184 Tiefenbach (DE)
(72) Inventor: Gassner, Paul, 84184 Tiefenbach (DE); Bauer, Florian, 84184 Tiefenbach (DE); Simmet, Kilian, 84184 Tiefenbach (DE)
(74) Representative: Engelhard, Markus

(57) **Abstract**

The present invention relates to the field of separating and/or enriching motile cells from other components of a cell sample. In particular, the present invention relates to a device for separating motile cells from other components of a cell sample, preferably for separating motile sperm cells from other components in a semen sample. The present invention further relates to a method for separating motile cells from other components of a cell sample using the device according to the present invention. The present invention further relates to a heating system for incubating said device. Moreover, the present invention relates to a device and its use for standardizing, structuring and supporting the workflow of separating and/or enriching motile cells.

## Description

### FIELD OF THE INVENTION

The present invention relates to the field of separating and/or enriching motile cells from other components of a cell sample. In particular, the present invention relates to a device for separating motile cells from other components of a cell sample, preferably for separating motile sperm cells from other components in a semen sample. The present invention further relates to a method for separating motile cells from other components of a cell sample using the device according to the present invention. The present invention further relates to a heating system for incubating said device. Moreover, the present invention relates to a device and its use for standardizing, structuring and supporting the workflow of separating and/or enriching motile cells.

### BACKGROUND OF THE INVENTION

The separation of motile cells from other components of a cell sample, such as non-motile cells and non-cellular, molecular components, is a critical process in various biological and medical applications. One of the most significant applications of this process is the separation of motile sperm cells from other components of a semen sample. The ability to efficiently isolate motile sperm cells is essential for assisted reproductive technologies (ART), including *in vitro* fertilization and artificial insemination, where the quality and motility of sperm are crucial determinants of success.

ART have become increasingly significant in addressing infertility issues worldwide. These technologies, which include *in vitro* fertilization and artificial insemination, provide hope for couples struggling to conceive naturally. The global importance of ART is underscored by its rapid growth and the substantial financial impact it has on the healthcare sector.

Over the past few decades, the use of ART has seen a considerable increase. According to the International Committee for Monitoring Assisted Reproductive Technologies (ICMART), in 2019 approximately 3.4 million ART cycles were performed globally each year, resulting in the birth of over 750,000 babies. This number continues to rise as more couples seek these treatments due to delayed childbearing, lifestyle factors, and increased awareness of fertility issues.

The financial importance of the ART sector is also noteworthy. The assisted reproductive technology market size was valued at USD 26.62 billion in 2023 and is projected to grow from USD 29.22 Billion in 2024 to USD 38.8 billion by 2032. This growth is driven by technological advancements, increased infertility rates, and supportive government initiatives in various countries.

In regions such as Europe and North America, ART services are well-established and continue to expand. In contrast, emerging markets in Asia-Pacific and Latin America are witnessing rapid growth due to increasing disposable incomes, improving healthcare infrastructure, and changing societal attitudes towards fertility treatments.

The importance of ART extends beyond financial metrics, as it plays a crucial role in addressing demographic challenges in countries with declining birth rates. By enabling couples to have children, ART contributes to population stability and supports the social and economic fabric of societies.

ART are not only crucial for human fertility treatments but also play a significant role in the agricultural sector, particularly in livestock management. The application of ART in agriculture has revolutionized animal breeding practices, leading to improved livestock productivity, genetic diversity, and overall herd health. One of the primary ART methods used in livestock is artificial insemination. By using artificial insemination, farmers can enhance the genetic quality of their herds without the need for natural mating, which can be logistically challenging and less efficient. Importantly, to increase the efficiency of artificial insemination isolated motile sperm cells with high quality and without other unwanted components are required.

The financial impact of ART in agriculture is substantial. According to a report by Grand View Research, the global veterinary artificial insemination market size was estimated at USD 7.37 billion in 2023 and is projected to grow at a CAGR of 6.34% from 2024 to 2030. This growth is driven by the increasing demand for high-quality animal products, advancements in ART techniques, and the rising adoption of these technologies by farmers worldwide. The use of ART in livestock also has significant implications for food security. By improving the productivity and efficiency of livestock breeding, ART contributes to the stable supply of animal products such as meat, milk, and eggs. This is particularly important in regions facing food scarcity and growing populations, where the demand for protein-rich foods is on the rise.

Isolating motile sperm cells is crucial for maximum efficiency of ART such as artificial insemination and *in vitro* fertilization since motility is one of the key markers indicating optimum functional condition and fertilization competence of a sperm. Motile sperm cells have a higher likelihood of reaching and penetrating the egg, thereby increasing the chances of conception. By selecting and using only the most viable, active, and functionally competent sperm cells, the overall success rates of ART procedures are significantly improved. This targeted approach minimizes the risk of failed fertilization attempts and enhances the safety, efficiency, and effectiveness of fertility treatments. Consequently, isolating motile sperm cells is a key factor in achieving better outcomes in ART.

Current and past techniques for separating motile cells often comprise a cell-permeable membrane through which motile cells should migrate. While these techniques aim to facilitate the migration of motile cells through the membrane, they are often plagued by several drawbacks that limit their effectiveness and scalability.

One significant disadvantage of current devices is the unfavorable or uneven pore distribution in the cell-permeable membrane. This inconsistency can lead to inefficient separation, as motile cells may be obstructed or unevenly distributed across the membrane surface. The uneven pore distribution can also result in areas of the membrane being underutilized, thereby reducing the overall efficiency of the separation process. This inefficiency is particularly problematic in clinical settings where the quality and purity of the separated motile cells are paramount.

Furthermore, the lack of structural support for the membrane often results in deformation, such as bending, expanding, or bulging, during the insertion or extraction of cell samples and conditioning media. Such deformations can compromise the integrity of the separation process and lead to suboptimal results. The deformation of the membrane can create areas of varying pore sizes, further exacerbating the issue of uneven pore distribution and leading to inconsistent separation outcomes.

Moreover, existing devices face limitations in scalability due to the lack of support for the membrane. This not only affects the scalability of the membrane itself but also restricts the overall scalability of the system. The inability to accommodate larger volumes in a sample chamber and/or a chamber for conditioning media, or to achieve a higher volume ratio between these chambers, further constrains the capacity of these devices to handle larger sample sizes or to be adapted for different applications. In research and clinical settings, the need to process varying sample sizes efficiently is crucial, and current devices fall short in this regard. In addition to this, existing devices do not comprise mechanisms or structures that prevent contamination of the sample or the conditioning media for separated motile cells. Thus, these systems are exposed to airborne germs and fungal spores.

Also, existing devices lack mechanisms or structures that prevent evaporation of liquid from the cell sample and/or the conditioning. This affects the quality of the separated motile cells or the reproducibility of test, since evaporation may lead to a change in osmolarity of the liquids, such as the conditioning media, during incubation of the existing devices.

Thus, there is a need for an improved device for separating motile cells from other components of a cell sample. Such a device should address the issues of uneven pore distribution, membrane deformation, and scalability limitations. By overcoming these challenges, the device would significantly enhance the efficiency and effectiveness of motile cell separation, providing valuable benefits for both research and clinical applications. Moreover, there is a need for an improved device that can separate motile cells, not only from non-motile or dead cells but also from other components that would act as disruptive factors in the further use or processing of separated motile cells. Furthermore, there is a need for a method of an improved separation of motile cells from other components of a cell sample, such as non-motile cells. Moreover, there is a need for a heating system that can further standardize and/or enhance the performance of the improved device for separating motile cells from other components of a cell sample.

### SUMMARY OF THE INVENTION

The present invention provides an improved device for separating motile cells from other components of a cell sample. Advantageously said device addresses the issues of uneven pore distribution, membrane deformation, and scalability limitations. Moreover, said device addresses the issues of liquid evaporation and contamination. By overcoming these challenges, the device significantly enhances the safety, efficiency and effectiveness of motile cell separation, providing valuable benefits for both research and clinical applications, in particular assisted reproductive technologies. Moreover, the improved device according to the present invention can separate motile cells, not only from non-motile or dead cells but also from other components that would act as disruptive factors in the further use or processing of separated motile cells. Furthermore, the present invention provides a method of improved separation of motile cells from other components of a cell sample, such as non-motile cells and other disruptive components. The present invention further provides a heating system that can further standardize and/or enhance the performance of the improved device for separating motile cells from other components of a cell sample and/or improve the safety of the procedure.

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a device for separating motile cells from other components of a cell sample, the device comprising a housing, which comprises:
1) a lower chamber which is open at the top and confined to the sides by side walls and to the bottom by a floor plate,
   wherein a bottom surface of the floor plate facing the outside is essentially planar,
   wherein a sample port connects the lower chamber to the outside,
   wherein the lower chamber is designed to accommodate a cell sample which may be inserted into, and/or extracted from, the lower chamber via the sample port, wherein the cell sample contains, or is suspected of containing, motile cells;
2) an upper chamber which is open at the bottom and confined to the sides by side walls and to the top by a roof,
   wherein an upper surface of the roof facing the outside is essentially planar,
   wherein within the area between the side walls, the upper chamber is arranged in form of a continuous circuitous flow path, preferably in form of a serpentine flow path,
   wherein on one end of the continuous circuitous path, preferably the serpentine flow path, the roof comprises a medium port that connects the upper chamber to the outside, and at the other end of the continuous circuitous path, the roof comprises a valve or slit that connects the upper chamber to the outside for pressure regulation;
   wherein the upper chamber is designed to accommodate a conditioning medium which may be introduced into, and/or extracted from, the upper chamber via the medium port;
   wherein the upper chamber is located on top of the lower chamber;
   wherein, when the lower chamber and the upper chamber are assembled in the housing, the side walls of the upper and lower chamber are aligned with each other so that the housing is closed, apart from the sample port of the lower chamber, the medium port of the upper chamber and the valve or slit for pressure regulation;
3) a cell-permeable membrane which is located between the lower chamber and the upper chamber, wherein the cell-permeable membrane is located on top of the lower chamber and below the upper chamber;
   and optionally
4) one or more removable lids, wherein the one or more removable lids are placed on the sample port of the lower chamber, the medium port of the upper chamber, and/or the valve or slit of the upper chamber;
   wherein the other components of the cell sample are selected from one or more of the group comprising non-motile cells, plasma components such as seminal plasma components, hormones, lipids, lipid compounds, dead cells, erythrocytes, leukocytes, precursor cells, other unwanted cells, proteins, enzymes, ions, minerals, sugars, vitamins, antioxidants, pro-inflammatory and anti-inflammatory molecules, nucleotides, nucleosides, contaminating microorganisms, bacteria, fungal spores, peptides, glycoproteins, metabolites, antimicrobial agents, growth factors, prostanoids, neurotransmitters, extracellular vesicles, nucleic acids, other small molecules, and cellular debris.

**In** one embodiment, the motile cells are selected from motile eukaryotic cells, such as motile mammalian sperm cells or motile avian sperm cells; preferably selected from motile sperm cells from a species of an order selected from the orders of primates, artiodactyla, perissodactyla, carnivora, cetacea, chiroptera, rodentia, accipitriformes, falconiformes, galliformes; more preferably selected from motile human sperm cells, bovine sperm cells, porcine sperm cells, equine sperm cells, caprine sperm cells, canine sperm cells, feline sperm cells, ovine sperm cells, dolphin sperm cells, bat sperm cells, rat sperm cells, hawk sperm cells, falcon sperm cells, and turkey sperm cells; even more preferably motile human sperm cells, bovine sperm cells, porcine sperm cells, and equine sperm cells; most preferably motile human sperm cells.

In one embodiment, the cell sample is a mammalian semen sample or an avian semen sample; preferably a semen sample from a species of an order selected from the orders of primates, artiodactyla, perissodactyla, carnivora, cetacea, chiroptera, rodentia, accipitriformes, falconiformes, galliformes; more preferably the cell sample is selected from a human semen sample, bovine semen sample, porcine semen sample, equine semen sample, caprine semen sample, canine semen sample, feline semen sample, ovine semen sample, dolphin semen sample, bat semen sample, rat semen sample, hawk semen sample, falcon semen sample, turkey semen sample; more preferably human semen sample, bovine semen sample, porcine semen sample, equine semen sample; most preferably a human semen sample;
wherein, further preferably, the seminal fluid is a native seminal fluid.

In one embodiment, the other components of the cell sample are selected from one or more of the group comprising non-motile sperm cells, seminal plasma components, sperm precursor cells, dead cells, unwanted cells, hormones, proteins and enzymes, ions, minerals, sugars, lipids, vitamins, other organic compounds, antioxidants, pro-inflammatory and anti-inflammatory molecules, nucleotides and nucleosides, contaminating microorganisms, bacteria, fungal spores, peptides, glycoproteins, metabolites, antimicrobial agents, growth factors, prostanoids, neurotransmitters, extracellular vesicles, nucleic acids, other small molecules, and cellular debris.

In one preferred embodiment, the other components are selected from one or more of the group comprising non-motile sperm cells, dead cells, seminal plasma components, sperm precursor cells, unwanted cells, hormones, lipids, proteins and enzymes, contaminating microorganisms, bacteria, and fungal spores.

In one preferred embodiment, the other components are selected from non-motile sperm cells, dead cells, seminal plasma components, contaminating microorganisms, bacteria, and fungal spores, more preferably non-motile sperm cells, dead cells, seminal plasma components.

In one embodiment, the continuous circuitous flow path, preferably the serpentine flow path of the upper chamber is formed by the side walls of the upper chamber and one or more inner walls within the upper chamber.

In one embodiment, the upper chamber in form of the continuous circuitous flow path, such as the serpentine flow path, allows for a gentle insertion and/or extraction of a conditioning medium, wherein, preferably, the insertion and/or extraction of the conditioning medium is air-bubble free.

In one embodiment, the continuous circuitous flow path, such as the serpentine flow path, of the upper chamber, preferably the inner walls of said upper chamber forming said continuous circuitous flow path, serves as support for the cell-permeable membrane, thereby stabilizing the cell-permeable membrane.

In one preferred embodiment, said support for the cell-permeable membrane prevents the cell-permeable membrane from deforming, such as bending, expanding and/or bulging, when the cell sample is inserted into, and/or extracted from, the lower chamber and/or when the conditioning medium is inserted into, and/or extracted from, the upper chamber; and/or
wherein, further preferably, said support for the cell-permeable membrane enables
larger volumes of the lower chamber and/or the upper chamber, and/or
a higher volume ratio of the lower chamber versus the volume of the upper chamber, and/or
a larger cell-permeable membrane, and/or
a larger surface area of the cell-permeable membrane, and/or
a higher ratio of surface area of the cell-permeable membrane to volume of the lower chamber and/or the upper chamber,
when compared to a device without an upper chamber in form of a continuous circuitous flow path, preferably a serpentine flow path.

In one embodiment, the volume of the lower chamber is in a range of about 500 µL to about 5 mL, preferably in a range of about 750 µL to about 4 mL, more preferably in a range of about 1 mL to about 3 mL, even more preferably in a range of about 1.5 mL to about 2.5 mL, most preferably the volume of the lower chamber is about 2 mL.

In one embodiment, the volume of the upper chamber is in a range of about 100 µL to about 2 mL, preferably in a range of about 400 µL to about 1.6 mL, more preferably in a range of about 600 µL to about 1.2 mL, even more preferably in a range of about 700 µL to about 1 mL, most preferably the volume of the upper chamber is about 850 µL.

In one embodiment, the volume of the lower chamber is larger than the one of the upper chamber, preferably by a factor in a range of about 1.2 to about 4, more preferably in a range of about 1.5 to about 3.5, even more preferably in a range of about 2 to about 3, even more preferably in a range of about 2 to about 2.5, most preferably by a factor of about 2.35.

In one embodiment, a larger volume of the lower chamber compared to the volume of the upper chamber increases the yield of motile cells, such as motile healthy sperm cells, and thus a greater selection efficiency
either when compared to a device in which the volumes of the upper chamber and the lower chamber is about similar, or in which the volume of the upper chamber is larger than the volume of the lower chamber,
or when compared to a device in which the volume of the lower chamber is not larger than the one of the upper chamber, preferably by a factor in a range of about 1.2 to about 4, more preferably in a range of about 1.5 to about 3.5, even more preferably in a range of about 2 to about 3, even more preferably in a range of about 2 to about 2.5, most preferably by a factor of about 2.35.

In one embodiment, the cell-permeable membrane is welded onto the side walls and the one or more inner walls of the upper chamber, thereby avoiding the use of potentially toxic adhesives.

In one embodiment, the cell-permeable membrane comprises pores with a pore size in the range of about 5 µm to about 12 µm, preferably in the range of about 5 µm to about 10 µm, more preferably in the range of about 5 µm to about 8 µm, most preferably a pore size of about 7 µm.

In one embodiment, the cell-permeable membrane is a monofilament fabric, preferably a monofilament fabric made of polyamide.

In one embodiment, the cell-permeable membrane is a track-etched membrane, preferably a track-etched membrane made of polyester.

In one embodiment, the cell-permeable membrane has an open area in a range of about 0.5% to about 20% of a total surface area of the cell-permeable membrane, preferably in a range of about 0.75% to about 15%, more preferably in a range of about 1% to about 10%.

In one embodiment, the cell-permeable membrane is a monofilament fabric with an open area in a range of about 0.5% to about 15% of the total surface area of the cell-permeable membrane, preferably in a range of about 0.75% to about 12%, more preferably in a range of about 1% to about 10%, even more preferably in a range of about 1% to about 5%, even more preferably in a range of about 1% to about 3%, most preferably with an open area of around 1%.

In one embodiment, the cell-permeable membrane is a track-etched membrane with an open area in a range of about 5% to about 20% of the total surface area of the cell-permeable membrane, preferably in a range of about 7.5% to about 15%, more preferably in a range of about 8% to about 12%, even more preferably in a range of about 9% to about 11%, most preferably with an open area of around 10%.

In one embodiment, the one or more removable lids prevent contamination of the conditioning medium in the upper chamber and/or the cell sample in the lower chamber.

In one embodiment, the one or more removable lids prevent evaporation of liquid from the lower chamber and/or the upper chamber.

In one aspect, the present invention relates to the use of the device according to the present invention for the separation of motile cells from other components of a cell sample, wherein the other components of the cell sample are selected from one or more of the group comprising non-motile cells, plasma components such as seminal plasma components, hormones, lipids, lipid compounds, dead cells, erythrocytes, leukocytes, precursor cells, other unwanted cells, proteins, enzymes, ions, minerals, sugars, vitamins, antioxidants, pro-inflammatory and anti-inflammatory molecules, nucleotides, nucleosides, contaminating microorganisms, peptides, glycoproteins, metabolites, antimicrobial agents, growth factors, prostanoids, neurotransmitters, extracellular vesicles, nucleic acids, other small molecules, and/or cellular debris;
preferably for the separation of motile sperm cells from other components selected from one or more of the group comprising non-motile sperm cells, seminal plasma components, sperm precursor cells, dead cells, unwanted cells, hormones, proteins and enzymes, ions and minerals, sugars, lipids, vitamins, other organic compounds, antioxidants, pro-inflammatory and anti-inflammatory molecules, nucleotides and nucleosides, contaminating microorganisms, erythrocytes, leukocytes, peptides, glycoproteins, metabolites, antimicrobial agents, growth factors, prostanoids, neurotransmitters, extracellular vesicles, nucleic acids, other small molecules, and/or cellular debris;
more preferably, for the separation of motile sperm cells from non-motile sperm cells, dead cells, seminal plasma components, sperm precursor cells, prostaglandins, and/or proteins.

In one embodiment, such use typically comprises the step of inserting a cell sample or adjusted cell sample into the lower chamber via the sample port using a syringe in a volume sufficient to completely fill the lower chamber.

In one aspect, the present invention relates to the use of the device according to the present invention for the selection and/or enrichment of motile cells from a cell sample, preferably for the selection and/or enrichment of motile sperm cells from a sperm cell sample.

In one embodiment, such use typically comprises the step of inserting a cell sample or adjusted cell sample into the lower chamber via the sample port using a syringe in a volume sufficient to completely fill the lower chamber.

In another aspect, the present invention relates to a method for separating motile cells from other components of a cell sample using the device according to the present invention, the method comprising the steps of
1) acquiring a cell sample which contains, or is suspected of containing, motile cells;
2) optionally, adjusting the volume of the cell sample with a sample buffer to a volume that equals at least the volume of the lower chamber, preferably to a volume that is about 5% to about 10% larger than the volume of the lower chamber, thereby resulting in an adjusted cell sample;
3) inserting the cell sample or the adjusted cell sample into the lower chamber via the sample port using a syringe in a volume sufficient to completely fill the lower chamber;
4) inserting a conditioning medium into the upper chamber via the medium port using a syringe in a volume sufficient to completely fill the upper chamber;
5) optionally, placing the one or more removable lids on the sample port, the medium port and/or the valve or slit;
6) incubating the device at a temperature in a range of about 20°C to about 40°C, preferably in a range of about 25°C to about 38°C, more preferably in a range of about 30°C to about 37°C, even more preferably in a range of about 35°C to about 37°C, most preferably at about 37°C, for a time period in a range of about 10 minutes to about 70 minutes; preferably in a range of about 15 minutes to about 60 minutes, more preferably in a range of about 30 minutes to about 45 minutes, wherein during incubation of the device, motile cells move upwards from the lower chamber containing the cell sample or adjusted cell sample through the cell-permeable membrane into the upper chamber containing the conditioning medium;
7) optionally, removing the one or more removable lids; and
8) extracting conditioning medium, preferably a conditioning medium containing, or suspected of containing, motile cells, from the upper chamber via the medium port using a syringe, wherein, preferably, a part of the volume of the conditioning medium is left in the upper chamber, wherein, more preferably about 60% to about 98% of the volume of the conditioning medium in the upper chamber is extracted, even more preferably about 65% to about 90% of the volume, even more preferably about 70% to about 80% of the volume; wherein the motile cells and/or the cell sample are as defined herein.

In one embodiment, incubating in step 6) is performed using a heating gradient; wherein, preferably, a temperature in the lower chamber is held lower than a temperature in the upper chamber.

In one preferred embodiment, the temperature in the lower chamber is held at a temperature in a range of about 30°C to about 37°C, preferably in a range of about 32°C to about 36°C, most preferably at about 35°C.

In one preferred embodiment the temperature in the upper chamber is held at a temperature in a range of about 35°C to about 40°C, preferably in a range of about 36°C to about 38°C, most preferably at about 37°C.

In one embodiment, the method further comprises the step of
9) storing the extracted conditioning medium until use.

In one aspect, the present invention relates to a heating system for incubating a device according to present invention, comprising a heating block with a heated lid, wherein the heating system comprises a space in which the device can be placed, so that when the heating system is closed, the heating block is below the lower chamber of the device and the heated lid is on top of the upper chamber of the device;
wherein the heating system further comprises a computer processor, a computer readable storage medium, and a user interface;
wherein the temperature of the heating block and the heated lid can be controlled separately;
wherein, preferably, the heated lid is in direct contact with the essentially planar upper surface of the roof of the upper chamber and/or the heating block is in direct contact with the essentially planar bottom surface of the floor plate of the lower chamber.

In one aspect, the present invention relates to the use of a heating system according to the present invention for separating motile cells from other components of a cell sample, wherein, preferably, the heating system is used in a method according to the present invention;
wherein, further preferably, during incubation of the device in step 6), the temperature of the heating block is set to a lower temperature than the temperature of the heated lid.

In one preferred embodiment, the temperature of the heating block is set to a temperature in a range of about 30°C to about 37°C, preferably in a range of about 32°C to about 36°C, most preferably to about 35°C.

In one preferred embodiment, the temperature of the heated lid is set to a temperature in a range of about 35°C to about 40°C, preferably in a range of about 36°C to about 38°C, most preferably to about 37°C.

In one aspect, the present invention relates to a kit for the separation of motile cells from other components of a cell sample, the kit comprising
a) a device according to the present invention; and
one or more of, optionally all of:
b) one or more syringes for inserting a cell sample into or extracting a cell sample from the lower chamber;
c) one or more syringes for inserting conditioning medium into and/or extracting conditioning medium from the upper chamber;
d) a sample buffer;
e) a conditioning medium;
f) a sterile collection container for the cell sample;
g) one or more sterile capillaries for aspirating and/or dispensing the cell sample and/or the conditioning medium into the corresponding syringes of b) and/or c); or
h) a heating system according to the present invention.

In one aspect, the present invention relates to the use of the device according to the present invention in a method of *in vitro* fertilization.

In one embodiment, the method comprises one or more, optionally all, of the steps of:
a) ovarian stimulation, preferably using hormonal medications to stimulate ovaries of a female patient to produce one or multiple eggs;
b) obtaining one or more eggs that are mature from the ovaries;
c) obtaining a semen sample from a male patient;
d) separating motile sperm cells from other components in said semen sample using the device according to the present invention and, optionally, the heating system according to the present invention;
e) fertilizing the obtained eggs with the separated motile sperm cells obtained in step d) to obtain one or more fertilized eggs; wherein, preferably, said obtained eggs are either mixed with said separated motile sperm cells in a laboratory dish to allow fertilization, or by intracytoplasmic sperm injection, where a separated motile sperm cell is injected directly into an egg;
f) culturing the one or more fertilized eggs using suitable culturing techniques to obtain one or more embryos;
g) transferring one or more embryos into the uterus of a biologically female patient, optionally the biologically female patient of step a);
h) optionally, monitoring success of the *in vitro* fertilization, preferably by performing a pregnancy test after a suitable amount of time.

In one aspect, the present invention relates to the use of the device according to the present invention in a method of artificial insemination.

In one embodiment, the method comprises one or more, optionally all, of the steps of:
a) monitoring a ovulation cycle of a female patient to determine the optimal time of insemination;
b) obtaining a semen sample from a male patient;
c) separating motile sperm cells from other components in said semen sample using the device according to the present invention and optionally the heating system according to the present invention;
d) inserting the separated motile sperm cells into the uterus of said biologically female patient;
e) optionally, monitoring success of the artificial insemination, preferably by performing a pregnancy test after a suitable amount of time.

### DETAILED DESCRIPTION

In one embodiment, the term "patient" relates to a human or an animal, preferably a human.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

In a first aspect, the present invention relates to a device for separating motile cells from other components of a cell sample, the device comprising a housing, which comprises:
1) a lower chamber which is open at the top and confined to the sides by side walls and to the bottom by a floor plate,
   wherein a bottom surface of the floor plate facing the outside is essentially planar,
   wherein a sample port connects the lower chamber to the outside,
   wherein the lower chamber is designed to accommodate a cell sample which may be inserted into, and/or extracted from, the lower chamber via the sample port, wherein the cell sample contains, or is suspected of containing, motile cells;
2) an upper chamber which is open at the bottom and confined to the sides by side walls and to the top by a roof,
   wherein an upper surface of the roof facing the outside is essentially planar,
   wherein within the area between the side walls, the upper chamber is arranged in form of a continuous circuitous flow path, preferably in form of a serpentine flow path,
   wherein on one end of the continuous circuitous path, preferably the serpentine flow path, the roof comprises a medium port that connects the upper chamber to the outside, and at the other end of the continuous circuitous path, the roof comprises a valve or slit that connects the upper chamber to the outside for pressure regulation;
   wherein the upper chamber is designed to accommodate a conditioning medium which may be introduced into, and/or extracted from, the upper chamber via the medium port;
   wherein the upper chamber is located on top of the lower chamber;
   wherein, when the lower chamber and the upper chamber are assembled in the housing, the side walls of the upper and lower chamber are aligned with each other so that the housing is closed, apart from the sample port of the lower chamber, the medium port of the upper chamber and the valve or slit for pressure regulation;
3) a cell-permeable membrane which is located between the lower chamber and the upper chamber, wherein the cell-permeable membrane is located on top of the lower chamber and below the upper chamber;
   and optionally
4) one or more removable lids, wherein the one or more removable lids are placed on the sample port of the lower chamber, the medium port of the upper chamber, and/or the valve or slit of the upper chamber;
   wherein the other components of the cell sample are selected from one or more of the group comprising non-motile cells, plasma components such as seminal plasma components, hormones, lipids, lipid compounds, dead cells, erythrocytes, leukocytes, precursor cells, other unwanted cells, proteins, enzymes, ions, minerals, sugars, vitamins, antioxidants, pro-inflammatory and anti-inflammatory molecules, nucleotides, nucleosides, contaminating microorganisms, bacteria, fungal spores, peptides, glycoproteins, metabolites, antimicrobial agents, growth factors, prostanoids, neurotransmitters, extracellular vesicles, nucleic acids, other small molecules, and cellular debris.

The term "essentially planar (surface)", as used herein, refers to a surface that is predominantly flat and level, with only minor deviations or irregularities that do not significantly affect its overall flatness or functionality for the intended purpose. For the present application, essentially planar with respect to the upper surface of the roof of the upper chamber that is facing the outside, may also refer to the surface of the roof that is predominantly flat and level, except for the medium port and/or the valve or slit for pressure regulation. Moreover, and essentially planar surface according to the present invention also refers to surfaces wherein the majority of the surface area is planar, wherein however, some parts of the surface area may deviate in some areas. This may, for example, be due to other structural components of the device, such as ports, slits, valves, recesses, protrusions, but also due to imprints on the surface that may be raised or recessed.

The term "outside", as used herein, refers to the surrounding environment of the device.

In one embodiment, the motile cells are selected from motile eukaryotic cells, such as motile mammalian sperm cells or motile avian sperm cells; preferably selected from motile sperm cells from a species of an order selected from the orders of primates, artiodactyla, perissodactyla, carnivora, cetacea, chiroptera, rodentia, accipitriformes, falconiformes, galliformes; more preferably selected from motile human sperm cells, bovine sperm cells, porcine sperm cells, equine sperm cells, caprine sperm cells, canine sperm cells, feline sperm cells, ovine sperm cells, dolphin sperm cells, bat sperm cells, rat sperm cells, hawk sperm cells, falcon sperm cells, and turkey sperm cells; even more preferably motile human sperm cells, bovine sperm cells, porcine sperm cells, and equine sperm cells; most preferably motile human sperm cells.

In one embodiment, the cell sample is a mammalian semen sample or an avian semen sample; preferably a semen sample from a species of an order selected from the orders of primates, artiodactyla, perissodactyla, carnivora, cetacea, chiroptera, rodentia, accipitriformes, falconiformes, galliformes; more preferably the cell sample is selected from a human semen sample, bovine semen sample, porcine semen sample, equine semen sample, caprine semen sample, canine semen sample, feline semen sample, ovine semen sample, dolphin semen sample, bat semen sample, rat semen sample, hawk semen sample, falcon semen sample, turkey semen sample; more preferably human semen sample, bovine semen sample, porcine semen sample, equine semen sample; most preferably a human semen sample;
wherein, further preferably, the seminal fluid is a native seminal fluid.

The term "native seminal fluid", as used herein, refers to the original, unaltered fluid produced by the male reproductive system, containing sperm cells and various other components.

In one embodiment, the other components of the cell sample are selected from one or more of the group comprising non-motile sperm cells, seminal plasma components, sperm precursor cells, dead cells, unwanted cells, hormones, proteins and enzymes, ions, minerals, sugars, lipids, vitamins, other organic compounds, antioxidants, pro-inflammatory and anti-inflammatory molecules, nucleotides and nucleosides, contaminating microorganisms, bacteria, fungal spores, peptides, glycoproteins, metabolites, antimicrobial agents, growth factors, prostanoids, neurotransmitters, extracellular vesicles, nucleic acids, other small molecules, and cellular debris.

In one embodiment, the proteins and enzymes are selected from prostate-specific antigen (PSA), acid phosphatase, semenogelin, fibronectin, lactoferrin, zinc-alpha-2-glycoprotein.

In one embodiment, the unwanted cells are selected from erythrocytes, leukocytes (such as neutrophils, lymphocytes, and macrophages), epithelial cells, cells from the lining of the reproductive tract and germ cells (such as spermatocytes and spermatids).

In one embodiment, the cellular debris is selected from membrane fragments and cytoplasmatic droplets.

In one embodiment, the ions and minerals are selected from zinc, calcium, magnesium, potassium, and sodium.

In one embodiment, the sugars are selected from fructose and glucose.

In one embodiment, the lipids are selected from cholesterol and phosphorolipids, and/or the vitamins are selected from vitamin C and vitamin B12.

In one embodiment, the other organic compounds are selected from amino acids, citric acids, urea, and creatine and/or the antioxidants are selected from glutathione and superoxide dismutase.

In one embodiment, the pro-inflammatory and anti-inflammatory molecules are selected from cytokines and chemokines.

In one embodiment, the nucleotides and nucleosides are selected from ATP, ADP, and AMP.

In one embodiment, the peptides are selected from endorphins and enkephalins.

In one embodiment, the glycoproteins are selected from prostate-specific membrane antigen (PSMA) and clusterin.

In one embodiment, the metabolites are selected from lactate and pyruvate, and/or the antimicrobial agents are selected from defensins and lysozyme.

In one embodiment, the growth factors are selected from epidermal growth factor (EGF), and transforming growth factor-beta (TGF-β), and/or the neurotransmitters are selected from leukotrienes and dopamine.

In one embodiment, the extracellular vesicles are selected from exosomes and microvesicles.

In one embodiment, the nucleic acids are selected from DNA including DNA fragments, and RNA including RNA fragments and microRNAs.

In one embodiment, the other small molecules are selected from nitric oxide and hydrogen peroxide.

In one preferred embodiment, the other components are selected from one or more of the group comprising non-motile sperm cells, dead cells, seminal plasma components, sperm precursor cells, unwanted cells, hormones, lipids, proteins and enzymes, contaminating microorganisms, bacteria, and fungal spores.

In one preferred embodiment, the other components are selected from non-motile sperm cells, dead cells, seminal plasma components, contaminating microorganisms, bacteria, and fungal spores, more preferably non-motile sperm cells, dead cells, seminal plasma components.

In preferred embodiments, the device according to the present invention not only separates motile cells from non-motile cells. Instead, the device of the present invention also separates motile cells from other components of a cell sample that may act as a disruptive factor in the further use of the motile cells. This retention function for the above components is of high importance for enhancing the further analysis and/or use of the motile cells. In case of the selection of motile sperm cell, this retention function for the above components is of high importance for enhancing the efficiency of assisted reproductive technologies, such as *in vitro* fertilization and artificial insemination.

The other components of a cell sample can act as disruptive factors in assisted reproductive technologies for at least the following reasons:
Non-motile sperm cells can interfere with the selection of healthy, motile sperm needed for successful fertilization, reducing the efficiency of the process.

With respect to seminal plasma components, while seminal plasma is essential for natural fertilization, its components can inhibit sperm function or interfere with the culture media used in *in vitro* fertilization.

Sperm precursor cells are not capable of fertilization and can contaminate the sample, making it harder to isolate viable sperm.

The presence of dead cells can release enzymes and other substances that may damage or inhibit the function of live sperm cells.

Unwanted cells include epithelial cells or leukocytes, which can introduce DNA or other contaminants that may affect the outcome of fertilization.

Hormones, proteins, and enzymes can alter the sperm's environment, potentially affecting motility and the ability to fertilize an egg.

In preferred embodiment, the hormones are selected from prostaglandins, testosterone, follicle-stimulating hormone (FSH), luteinizing hormone (LH), even more preferably prostaglandins.

The term "prostaglandins", as used herein refers to a group of physiologically active lipid compounds that have diverse hormone-like effects in animals. In the context of assisted reproductive technologies such as *in vitro* fertilization and artificial insemination, prostaglandins can be disruptive factors for several reasons. Prostaglandins can affect the motility of sperm cells. While some prostaglandins may enhance motility, others can inhibit it, leading to inconsistent and unpredictable effects on the sperm's ability to reach and fertilize the egg. Prostaglandins are known to induce uterine contractions. In the context of artificial insemination, this can lead to premature expulsion of the sperm from the reproductive tract, reducing the chances of successful fertilization. Prostaglandins are involved in the inflammatory response. Their presence can lead to an inflammatory environment in the reproductive tract, which can be detrimental to both sperm and egg cells, potentially reducing the chances of successful fertilization and implantation. Sperm capacitation is a process that sperm must undergo to gain the ability to penetrate and fertilize an egg. Prostaglandins can influence this process, potentially leading to either premature capacitation or failure to capacitate, both of which can reduce the likelihood of successful fertilization. Prostaglandins can interact with other cells and molecules in the reproductive tract, potentially leading to the release of other factors that can be harmful to sperm cells or interfere with the fertilization process. Prostaglandins can also affect the quality of the oocyte (egg cell). An inflammatory environment or altered biochemical conditions due to prostaglandins can negatively impact the oocyte, reducing its viability and the chances of successful fertilization and embryo development.

The removal of ions, minerals, sugars, lipids, vitamins, and/or other organic compounds is also highly relevant, as imbalances or excesses of these substances can affect sperm viability and function.

Concerning antioxidants, pro-inflammatory and anti-inflammatory molecules, while antioxidants can protect sperm, imbalances in inflammatory molecules can damage sperm or affect their function.

Nucleotides and nucleosides can interfere with the genetic material of sperm, potentially leading to abnormalities.

Contaminating microorganisms, bacteria, and fungal spores can introduce infections or compete with sperm for nutrients, affecting their viability.

Peptides, glycoproteins, and metabolites can alter the biochemical environment, potentially affecting sperm motility and function.

Antimicrobial agents, while intended to kill microorganisms, these agents can also harm sperm cells.

Growth factors and prostanoids can alter cellular signaling pathways, affecting sperm function.

Neurotransmitters can affect sperm motility and the ability to fertilize an egg.

Extracellular vesicles and nucleic acids can carry signals or genetic material that may interfere with normal sperm function.

Other small molecules and cellular debris can physically obstruct sperm or alter the chemical environment, affecting sperm health and function.

Each of these components can introduce variables that complicate the isolation and use of healthy sperm cells, thereby potentially reducing the success rates of assisted reproductive technologies. Thus, at least for the above reasons, the separation of motile cells, in particular motile sperm cells, from the components described above is of high importance. The device of the present invention in capable of selecting motile cells from said other components in a cell sample.

The term "separation of motile cells", as used herein, refers to separation, purification and/or enrichment of motile cells from a cell sample that may also comprise other, potentially disruptive components. According to the present invention, separation of motile cells is achieved by the spatial arrangement of the lower chamber, the upper chamber and the cell-permeable membrane on the one hand and the pore size of the cell-permeable membrane on the other hand. Thus, separation is achieved by the ability of motile cells to move upwards from the lower chamber through the membrane into the upper chamber, thereby overcoming the force of gravity. Importantly, said motile cells further need to fulfill the size requirements to pass through the pores of the cell-permeable membrane. Other non-motile and/or too large cells or components cannot pass the cell-permeable membrane and are retained in the lower compartment. Separation of motile cells, as used herein, may also be defined by the retention of other (unwanted) components as defined herein, wherein, preferably at least 60% of either one particular other component or one or more other components, optionally all other components as defined herein, are retained by the cell-permeable membrane, more preferably at least 70%, even more preferably at least 80%, even more preferably at least 90%, even more preferably at least 95%, even more preferably at least 97%, most preferably at least 99% of either one particular other component or one or more other components, optionally all other components as defined herein, are retained by the cell-permeable membrane.

The term "continuous circuitous flow path", as used herein, refers to a fluid flow route that is designed to be unbroken and looping, allowing fluid to travel in a closed or semi-closed loop without interruption. This type of flow path is engineered to maintain consistent fluid motion and minimize dead zones. The circuitous nature of the path can help in maintaining uniform flow rates and pressures, when medium is inserted into and/or extracted from the upper chamber via the medium port. The looping nature of the flow path minimizes the presence of dead zones where fluid movement is minimal or absent. By providing a smooth and continuous flow, the circuitous path reduces the risk of mechanical stress and shear forces that can occur in systems without said continuous circuit flow path. This gentle handling is essential for preserving the integrity and functionality of motile sperm cells, thus, the risk of cell damage is reduced.

The term "serpentine flow path", as used herein, refers to a fluid flow route characterized by its winding, snake-like pattern, which forces the fluid to traverse one or multiple turns and bends.

The term "unwanted cells", as used herein, refers to cells present in a sample that are not desired for a particular application or process and may need to be removed or separated from the target cells.

The term "contaminating microorganisms", as used herein, refers to unwanted bacteria, viruses, fungi, or other microorganisms that are present in a sample and may interfere with the intended use or analysis of the sample.

The term "cellular debris", as used herein, refers to fragments or remnants of cells, including dead cells, cell parts, and other particulate matter resulting from cell damage or death.

The term "designed to accommodate", as used herein, refers to the intentional configuration or construction of a device, component, or system to fit or hold a substance such as a cell sample or a conditioning medium.

The term "conditioning medium", as used herein, refers to a fluid or substance used to prepare, treat, or maintain the environment for cells or biological samples, ensuring optimal conditions for their viability, function, or processing. Depending on the respective motile cell to be separated, the skilled person will know which conditioning medium is suitable to be uses. The conditioning medium may either be a commercially available medium or a customized conditioning medium.

The term "sample buffer", as used herein, refers to a buffer suitable for dilution of a cell sample. Use of this buffer may be necessary if the volume of the sample is not sufficient to entirely fill the lower chamber. Depending on the respective motile cell to be separated, the skilled person will know which sample buffer is suitable to be used.

The term "cell-permeable membrane", as used herein, refers to a selective barrier that allows certain cells or molecules to pass through while restricting the movement of other components. Preferred materials for the cell-permeable membranes are a monofilament fabric, preferably a monofilament fabric made of polyamide, or track-etched membranes.

The term "volume sufficient to completely fill (a chamber)", as used herein, refers to the quantity of a fluid or substance required to completely occupy the internal space of a specified chamber, such as the upper chamber or the lower chamber of the device according to the present invention, preferably without leaving any voids.

In one embodiment, a cell sample may be introduced into and/or extracted from the lower chamber via the sample port.

In one embodiment, an upper surface area of the cell-permeable membrane faces the upper chamber, and a lower surface area of the cell-permeable membrane faces the lower chamber.

In one embodiment, the continuous circuitous flow path, preferably the serpentine flow path of the upper chamber is formed by the side walls of the upper chamber and one or more inner walls within the upper chamber.

The term "inner walls within the upper chamber", as used herein, refers to walls that are positioned within the area between the side walls that confine the upper chamber to the sides. In preferred embodiments, the inner walls of the upper chamber participate in forming the continuous circuitous flow path, preferably the serpentine flow path, of the upper chamber. Preferably, the inner walls are connected to the roof of the upper chamber. Further preferably, the inner walls of the upper chamber are the same height as the side walls of the upper chamber. Optionally, the inner walls are connected with each other and/or one or more of the sidewalls.

In one embodiment, the continuous circuitous flow path, such as the serpentine flow path, of the upper chamber, preferably the inner walls of said upper chamber forming said continuous circuitous flow path, serves as support for the cell-permeable membrane, thereby stabilizing the cell-permeable membrane.

In one embodiment, said support for the cell-permeable membrane prevents the cell-permeable membrane from deforming, such as bending, expanding and/or bulging, when the cell sample is inserted into, and/or extracted from, the lower chamber and/or when the conditioning medium is inserted into, and/or extracted from, the upper chamber; and/or
wherein, further preferably, said support for the cell-permeable membrane enables
larger volumes of the lower chamber and/or the upper chamber, and/or
a higher volume ratio of the lower chamber versus the volume of the upper chamber, and/or
a larger cell-permeable membrane, and/or
a larger surface area of the cell-permeable membrane, and/or
a higher ratio of surface area of the cell-permeable membrane to volume of the lower chamber and/or the upper chamber,
when compared to a device without an upper chamber in form of a continuous circuitous flow path, preferably a serpentine flow path.

In one embodiment, said support for the cell-permeable membrane stabilizes the volume of the upper chamber and lower chamber. Advantageously, said stabilization of the volumes may enhance the quality and/or reproducibility of the separation of motile cells from other components within a cell sample.

Supporting the cell-permeable membrane from the top, i.e. from within the upper chamber is advantageous when compared for example with a stabilization of a membrane from below the cell-permeable membrane. One advantage of this setup or design is that the volume of the upper chamber is directly stabilized. A further advantage of this setup is that membrane stabilization in general is optimized. When using the device for separating motile cells from other components of a cell sample, inserting of a cell sample or adjusted cell sample into the lower chamber via the sample port is performed prior to the insertion of the conditioning medium in the upper chamber. Thus, without support from the top, during this process the cell-permeable can easily deform, such as bend, expand and/or bulge, towards the upper chamber, which is empty at this point. The support from the upper side of the cell-permeable membrane directly prevents such deformation towards the upper chamber.

In one embodiment, a larger volume of the lower chamber compared to the upper chamber enables a more efficient use of a cell sample, such as a semen sample, by reducing the waste/loss of excess semen sample, that could otherwise not be analyzed or used. Thus, the larger volume of the lower chamber enables the use of an entire semen sample. Thus, by the larger volume of the lower chamber, a higher number of motile cells, such as sperm cells are available for the selection process.

In one embodiment, the upper chamber in form of the continuous circuitous flow path, such as the serpentine flow path, allows for a gentle insertion and/or extraction of a medium, such as the conditioning medium; wherein, preferably, the insertion and/or extraction of the medium is air-bubble free.

The term "air-bubble free", as used herein, refers to a condition where a fluid or medium contains no visible air bubbles or only an insignificant number of air bubbles that do not affect the intended function or quality of the medium.

The term "gentle insertion and/or extraction of a medium", as used herein, refers to the careful and controlled introduction or removal of a fluid or substance in a manner that minimizes turbulence, mechanical stress including shear forces, and disturbance to the biological specimens, ensuring their structural and functional integrity is maintained.

In preferred embodiments, this gentle insertion and/or extraction improves the quality of the separated motile cells, preferably the separated motile sperm cells. Gentle handling of sperm cells, including avoiding shear forces and air bubbles, is crucial for maintaining good sperm quality for several reasons:
Prevention of physical damage: Sperm cells are delicate and can be easily damaged by mechanical forces. Shear forces, which occur when cells are subjected to rapid or turbulent movement, can cause physical damage to the cell membrane and internal structures, leading to reduced motility and viability.

Maintenance of membrane integrity: The integrity of the sperm cell membrane is essential for its function, including motility and the ability to fertilize an egg. Gentle handling helps preserve the membrane's integrity, ensuring that the sperm cells remain functional.

Avoidance of oxidative stress: Air bubbles can introduce oxygen into the medium, leading to the generation of reactive oxygen species (ROS). ROS can cause oxidative stress, which damages sperm DNA, proteins, and lipids, ultimately reducing sperm quality and fertilization potential. Moreover, when air bubbles come into contact with biological specimens, such as sperm cells, the mechanical interaction at the interface can generate shear forces. These forces can cause physical damage to the cells, affecting their viability and function.

Preservation of motility: Sperm motility is a critical factor in successful fertilization. Gentle handling minimizes the risk of mechanical damage that can impair the sperm's ability to swim effectively, ensuring that a higher proportion of sperm cells retain their motility.

Reduction of apoptosis: Mechanical stress and oxidative damage can trigger apoptosis (programmed cell death) in sperm cells. By handling sperm gently and avoiding air bubbles, the likelihood of inducing apoptosis is reduced, leading to a higher number of viable sperm cells.

Prevention of clumping: Air bubbles and shear forces can cause sperm cells to clump together, making it difficult to isolate individual motile sperm for assisted reproductive techniques. Gentle handling helps maintain a uniform suspension of sperm cells, facilitating their selection and use.

Minimization of temperature Fluctuations: Gentle handling helps maintain a stable temperature environment for sperm cells. Rapid movements and the introduction of air bubbles can cause temperature fluctuations, which can negatively impact sperm function and viability.

Enhancement of fertilization potential: Overall, gentle handling practices help preserve the structural and functional integrity of sperm cells, enhancing their fertilization potential. This is particularly important in assisted reproductive technologies, where the quality of sperm directly influences the success rates of procedures like *in vitro* fertilization and artificial insemination.

In summary, gentle handling of sperm cells, avoiding shearing forces and air bubbles, is essential for maintaining sperm quality by preventing physical damage, preserving membrane integrity, reducing oxidative stress, maintaining motility, preventing apoptosis, avoiding clumping, minimizing temperature fluctuations, and ultimately enhancing fertilization potential. This gentle handling is made possible by the unique form of the continuous circuitous flow path of the upper chamber.

In one embodiment, the volume of the lower chamber is in a range of about 500 µL to about 5 mL, preferably in a range of about 750 µL to about 4 mL, more preferably in a range of about 1 mL to about 3 mL, even more preferably in a range of about 1.5 mL to about 2.5 mL, most preferably the volume of the lower chamber is about 2 mL.

In one embodiment, the volume of the upper chamber is in a range of about 100 µL to about 2 mL, preferably in a range of about 400 µL to about 1.6 mL, more preferably in a range of about 600 µL to about 1.2 mL, even more preferably in a range of about 700 µL to about 1 mL, most preferably the volume of the upper chamber is about 850 µL.

In one embodiment, the volume of the lower chamber is larger than the one of the upper chamber, preferably by a factor in a range of about 1.2 to about 4, more preferably in a range of about 1.5 to about 3.5, even more preferably in a range of about 2 to about 3, even more preferably in a range of about 2 to about 2.5, most preferably by a factor of about 2.35.

In one embodiment, a larger volume of the lower chamber compared to the volume of the upper chamber increases the yield of motile cells, such as motile healthy sperm cells, and thus a greater selection efficiency
when compared to a device in which the volumes of the upper chamber and the lower chamber is about similar, or in which the volume of the upper chamber is larger than the volume of the lower chamber,
or when compared to a device in which the volume of the lower chamber is not larger than the one of the upper chamber, preferably by a factor in a range of about 1.2 to about 4, more preferably in a range of about 1.5 to about 3.5, even more preferably in a range of about 2 to about 3, even more preferably in a range of about 2 to about 2.5, most preferably by a factor of about 2.35.

In one embodiment, the cell-permeable membrane is welded onto the side walls and the one or more inner walls of the upper chamber, thereby avoiding the use of potentially toxic adhesives.

Said potentially toxic adhesives can decrease the quality of the separated motile cells.

In one embodiment, the cell-permeable membrane comprises pores with a pore size in the range of about 5 µm to about 12 µm, preferably in the range of about 5 µm to about 10 µm, more preferably in the range of about 5 µm to about 8 µm, most preferably a pore size of about 7 µm.

In one embodiment, the cell-permeable membrane is a monofilament fabric, preferably a monofilament fabric made of polyamide.

In one embodiment, the cell-permeable membrane is a track-etched membrane, preferably a track-etched membrane made of polyester.

In one embodiment, the cell-permeable membrane has an open area in a range of about 0.5% to about 20% of a total surface area of the membrane, preferably in a range of about 0.75% to about 15%, more preferably in a range of about 1% to about 10%.

In one embodiment, the cell-permeable membrane is a monofilament fabric with an open area in a range of about 0.5% to about 15% of the total surface area of the membrane, preferably in a range of about 0.75% to about 12%, more preferably in a range of about 1% to about 10%, even more preferably in a range of about 1% to about 5%, even more preferably in a range of about 1% to about 3%, most preferably with an open area of around 1%.

In one embodiment, the cell-permeable membrane is a track-etched membrane with an open area in a range of about 5% to about 20% of the total surface area of the membrane, preferably in a range of about 7.5% to about 15%, more preferably in a range of about 8% to about 12%, even more preferably in a range of about 9% to about 11%, most preferably with an open area of around 10%.

The term "monofilament fabric", as used herein, refers to a type of membrane material made from single, continuous strands of fiber woven or knitted together to form a porous structure that allows selective permeability.

The term "track-etched membrane", as used herein, refers to a type of membrane created by irradiating a polymer film with charged particles and then chemically etching the tracks to form uniform, cylindrical pores, wherein, preferably said polymer film is made from polyester material.

The term "open area of a (cell-permeable) membrane", as used herein, refers to the proportion of the membrane surface that consists of pores or openings, allowing the passage of fluids or cells through the membrane.

In one preferred embodiment, the cell-membrane is not a polycarbonate membrane.

In one embodiment, the cell-permeable membrane exhibits a better, i.e. more uniform pore distribution compared to a polycarbonate cell-permeable membrane.

In one embodiment, the one or more removable lids prevent contamination of the conditioning medium in the upper chamber and/or the cell sample in the lower chamber.

In one embodiment, the one or more removable lids prevent evaporation of liquid from the lower chamber and/or the upper chamber.

In one embodiment, the each of the one or more removable lids is structurally adapted to fit to at least one of the sample port of the lower chamber, the medium port of the upper chamber, and/or the valve or slit of the upper chamber.

In one embodiment, one or more, optionally all of the sample port of the lower chamber, the medium port of the upper chamber, and/or the valve or slit of the upper chamber have a corresponding lid that exclusively can be placed on said sample port of the lower chamber, the medium port of the upper chamber, and/or the valve or slit of the upper chamber.

In one preferred embodiment, two or more, optionally all, of the one or more removable lids are combined to a combined lid that can be simultaneously placed on or removed from at least two of the sample port of the lower chamber, the medium port of the upper chamber, and/or the valve or slit of the upper chamber.

Advantageously, a combined lid enables the simultaneous covering of the at least two of the sample port of the lower chamber, the medium port of the upper chamber, and/or the valve or slit of the upper chamber. This simplifies and speeds up the handling of the lid and reduces the number of parts in particular small parts as individual lids. Moreover, the combined lid has haptic advantages as it is easier to be grabbed by a person working with the device of the present invention. Also, the reduced number of parts reduces the risk of mismatching lids to the wrong devices in case several devices are used at the same time.

In one embodiment, the one or more removable lids are individual lids that may be individually placed on or removed from the sample port of the lower chamber, the medium port of the upper chamber, and/or the valve or slit of the upper chamber.

In one preferred embodiment, the device further comprises a labeling area on or at the device for labeling with a sample identifier and/or subject identifier and/or date; wherein, preferably said labeling area is color coded (such as blue, yellow, red, green, orange, purple, grey, white, brown,...), wherein, even more preferably, said color coding allows for an easier and/or simpler identification of the respective sample.

The term "color coded", as used herein (for example in "labeling area is color coded"), refers to a system of using distinct colors to label or mark samples in order to easily and quickly identify and differentiate them based on their color. Advantages of color coding of labeling areas include, but are not limited to, enhanced visual identification, as color coding allows for immediate visual recognition of different samples, reducing the likelihood of errors in sample handling and identification. Moreover, color coding enables an improved organization. Using colors to categorize samples helps in organizing and managing multiple samples efficiently, making it easier to locate and retrieve specific samples when needed. Color coding further enhances time efficiency and leads to error reduction. The use of color-coded identifiers speeds up the process of identifying and sorting samples, saving valuable time in laboratory and clinical settings. By providing a clear and distinct method of identification, color coding reduces the risk of mislabeling or mixing up samples, thereby improving the accuracy and reliability of experimental or diagnostic results. Furthermore, color coding enables a simplified communication, since color-coded samples facilitate clear and straightforward communication among laboratory personnel, as colors can be easily described and recognized. Thus, the use of distinct colors to label samples for easy identification, offering advantages such as enhanced visual identification, improved organization, time efficiency, error reduction, and simplified communication.

In preferred embodiments, the sidewalls of the upper chamber and the lower chamber form an essentially oval or a circle, preferably an oval, even more preferably an ellipse, when viewed from the top.

In preferred embodiments, the upper chamber and the lower chamber are in an oval shape or a circle, preferably an oval shape, even more preferably an ellipse, when viewed from the top.

The term "oval", as used herein, refers to an essentially elliptical shape, preferably an elliptical shape. Such shape can be defined by its major and minor axes, which are the longest and shortest diameters of the ellipse, respectively. The major axis equals the longest diameter of the ellipse, passing through its center and both foci. The minor axis represents the shortest diameter of the ellipse, perpendicular to the major axis at the center.

An oval, preferably elliptic, form and/or shape of the lower chamber and the upper chamber offers several advantages. The oval shape can provide better support and distribution of forces across the cell-permeable membrane. This enhanced stability can prevent sagging or deformation of the cell-permeable membrane, ensuring consistent and reliable separation of motile cells from other components. Thus, the oval shape can enhance the stability of the membrane either separately and/or additionally to the stability provided by the inner walls of the upper chamber. Due to the improved stability of the cell-permeable membrane in an oval-shaped device, it is possible to design larger devices without compromising the integrity of the membrane. This allows for the processing of larger sample volumes, increasing the efficiency and throughput of cell separation, where necessary. Moreover, the elongated nature of an oval shape can promote more uniform flow dynamics within the chambers. In addition to this, the oval shape can distribute mechanical stresses more evenly across the device, reducing the risk of cracking or breaking under pressure. As used herein, the oval, preferably elliptic, form and/or shape refers to an essentially oval or an essentially elliptic form or shape. That form or shape may deviate in some areas, for example due to other structural components of the device.

In one preferred embodiment, the side walls of the upper chamber and the lower chamber form an oval, wherein the ratio of semi-major axis to semi-minor axis within said oval is in a range from about 1.2 : 1 to about 3 : 1, preferably in a range of about 1.3 : 1 to about 2.5 : 1, more preferably in a range of about 1.4 : 1 to about 2 : 1, even more preferably in a range of about 1.5 : 1 to about 1.9 : 1.

In one preferred embodiment, the ratio of semi-major axis to semi-minor axis is about 1.7 : 1.

In one embodiment, the continuous circuitous flow path, preferably the serpentine flow path, has a total of about 1 to about 101 turns, preferably of about 1 to about 91 turns, even more preferably of about 1 to about 81 turns, even more preferably of about 1 to about 71 turns, even more preferably of about 1 to about 61 turns, even more preferably of about 1 to about 51 turns, even more preferably of about 1 to about 41 turns, even more preferably of about 1 to about 31 turns, even more preferably of about 1 to about 21 turns, even more preferably of about 1 to 11 turns, even more preferably about 1 to 9 turns, even more preferably about 3 to 7 turns, even more preferably 5 turns.

In one embodiment, the continuous circuitous flow path, preferably the serpentine flow path of the upper chamber is formed by the side walls and about 1 to about 101 inner walls, preferably about 1 to about 91 inner walls, even more preferably about 1 to about 81 inner walls, even more preferably about 1 to about 71 inner walls, even more preferably about 1 to about 61 inner walls, even more preferably about 1 to about 51 inner walls, even more preferably about 1 to about 41 inner walls, even more preferably about 1 to about 31 inner walls, even more preferably about 1 to about 21 inner walls, even more preferably 1 to 11 inner walls.

In one embodiment, the continuous circuitous flow path, preferably the serpentine flow path, of the upper chamber is formed by the side walls and one or more inner walls within the upper chamber, wherein, preferably, the one or more inner walls comprise one central inner wall, which is arranged along the axis (in case of an oval along the major axis) of the upper chamber, and/or optionally, about 1 to 50 parallel inner walls on each side of the central inner wall (i.e. a total of 2 to 100 parallel inner walls and a total of 1 to 101 inner walls), preferably about 1 to about 45 parallel inner walls, more preferably about 1 to about 40 parallel inner walls, even more preferably about 1 to about 35 parallel inner walls, even more preferably about 1 to about 30 parallel inner walls, even more preferably about 1 to about 25 parallel inner walls, even more preferably about 1 to about 20 parallel inner walls, even more preferably about 1 to about 15 parallel inner walls, even more preferably about 1 to about 10 parallel inner walls, even more preferably about 1 to about 5 parallel inner walls.

In one preferred embodiment, one or more of the inner walls of the upper chamber are curved.

In one embodiment, the parallel inner walls of the upper chamber are essentially curved in form of concentric ellipses.

Advantageously, a curved shape of one or more of the inner walls prevents and/or reduced physical stress for the cells and other components when medium is inserted into and/or extracted from the upper chamber. Thus, the curved shape contributes to the fact that the separated motile cells can be gently extracted from the upper chamber of the device.

In one preferred embodiment, the inner walls of the upper chamber are connected to the roof of the upper chamber.

In one embodiment, the continuous circuitous flow path winds essentially parallel to or along the major axis of the shape of the upper chamber, i.e. the continuous circuitous flow path winds essentially perpendicular the minor axis.

In one embodiment, the continuous circuitous flow path winds essentially perpendicular to the major axis of the shape of the upper chamber, i.e. the continuous circuitous flow path winds essentially parallel to or along the minor axis.

In one embodiment, the width of the continuous circuitous flow path is in a range of about 0.2 mm to about 20 mm, preferably in a range of about 0.3 mm to about 15 mm, more preferably in a range of about 0.5 mm to about 10 mm; even more preferably in a range of about 1 mm to about 8 mm; even more preferably in a range of about 2 mm to about 6 mm; even more preferably in a range of about 3 mm to about 5 mm; most preferably the width of the continuous circuitous flow path is about 4 mm;
wherein optionally, the width of the continuous circuitous flow path can differ within one continuous circuitous flow path.

In one embodiment, an end of each inner wall, that is facing a turn of the continuous circuitous flow path is rounded, preferably to avoid sheering of cells at said ends.

In one embodiment, the distance between the inner walls and/or the inner walls and the side walls comprises a maximum of about 2 cm, preferably a maximum of about 1.5 cm, even more preferably a maximum of about 1 cm, even more preferably a maximum of about 0.8 cm, even more preferably a maximum of about 0.6 cm, most preferably a maximum of about 0.4 cm.

In one embodiment, the inner walls have a thickness in a range of about 0.1 mm to about 10 mm; preferably in a range of about 0.2 mm to about 9 mm, even more preferably in a range of about 0.3 mm to about 8 mm, even more preferably in a range of about 0.4 mm to about 7 mm, even more preferably in a range of about 0.5 mm to about 6 mm, even more preferably in a range of about 0.5 mm to about 5 mm, even more preferably in a range of about 0.5 mm to about 4 mm, even more preferably in a range of about 0.5 mm to about 3 mm, even more preferably in a range of about 0.5 mm to about 2 mm, even more preferably in a range of about 0.5 mm to about 1.5 mm, most preferably about 1 mm.

In one preferred embodiment, the side walls and the floor plate of the lower chamber are part of a base unit, wherein the base unit comprises
i) outer walls
ii) a horizontal platform at the upper end of the outer walls
iii) a groove in said horizontal platform; wherein, preferably, the groove has the same general shape as the upper chamber and the lower chamber when viewed from top;
iv) inner walls of the base unit within said groove, wherein said inner walls extend from the horizontal platform downwards, wherein the lower end of the inner walls of the base unit are connected to the upper end of the side walls of the lower chamber.

In one embodiment, the inner walls of the base unit within said groove comprise a horizontal inwards protruding ring at the bottom, and the side walls of the lower chamber comprise a horizontal outwards protruding ring at the top, wherein the inwards protruding ring of the side walls is directly connected to the outwards protruding ring of the lower chamber.

The term "ring", as used herein, does not only refer to a circular ring structure, but also encompasses ring structures that are oval or elliptic.

In one embodiment, the outer walls of the base unit starting from the lower end to the upper end are angled inwards (i.e. towards the center of the base unit), preferably with angle in a range of about 1° to about 45°, more preferably with an angle in a range of about 2° to about 20°, more preferably with an angle in a range of about 2° to about 15°, more preferable with an angle in a range of about 2° to about 10°, more preferable with an angle in a range of about 2° to about 5°, most preferably with an angle of about 3°.

Preferably, this enhances the stability of the device.

The term "inwards", as used herein, e.g. in "angled inwards", refers to the orientation towards the center of the device.

The term "outwards", as used herein, e.g. in "angled outwards", refers to the orientation away from the center of the device.

In one embodiment, the inner walls of the base unit within said groove starting from the lower end to the upper end are angled outwards (i.e. away from the center of the base unit), preferably with angle in a range of about 1° to about 45°, more preferably with an angle in a range of about 2° to about 20°, more preferably with an angle in a range of about 2 to
about 15°, more preferable with an angle in a range of about 2° to about 10°, more preferable with an angle in a range of about 2° to about 5°, most preferably with an angle of about 3°.

In one embodiment, when the shape of the lower chamber and the upper chamber is an oval or an ellipse, the groove in the horizontal platform is also oval or elliptic, wherein the oval of the groove and the oval of the upper chamber are concentric ellipses that share the same center.

In one embodiment, the side walls of the upper chamber, the inner walls of the upper chamber, and the roof of the upper chamber are part of an insertion unit, wherein said insertion unit comprises outer walls located at the top of the roof of the upper chamber, wherein said insertion unit, in particular said outer walls of the insertion unit are structurally adapted to sit within the groove of the horizontal platform of the base unit, further wherein, when said insertion unit is inserted into the groove of the base unit, the lower chamber and the upper chamber are assembled in the housing and the side walls of the upper and lower chamber are aligned with each other so that the housing is closed, apart from the sample port of the lower chamber, the medium port of the upper chamber and the valve or slit for pressure regulation.

In some embodiments, the inner walls and/or the horizontal platform of the base unit have one or more recesses (such as holes, mortises, or slots), and the outer walls of the insertion unit has protrusions at the corresponding positions (such as "latching noses", pins, tenons, or lugs), so that when said insertion unit is inserted into the groove of the base unit, the connection between the insertion unit and the base unit is stabilized.

In preferred embodiments, the sample port runs through the insertion unit, optionally the upper chamber, to connect the lower chamber with the outside at the top of the device.

In one embodiment, the sample port and/or the medium port are oriented vertically.

In one embodiment, the sample port and/or the medium port are oriented perpendicular to the lower chamber/and or the upper chamber.

In one embodiment, the sample port and/or the medium port are angled, preferably in an angle in a range of about 5° to about 80° with respect to a vertical axis which runs perpendicular to the device. More preferably, the sample port and/or the medium port are angled, in an angle in a range of about 10° to about 70°, even more preferably in a range of about 20° to about 60°, even more preferably in a range of about 30° to about 50°. A sample port and/or a medium port that is angled can further enhance gentle handling of the cell sample and/or the conditioning medium, preferably a conditioning medium containing, or suspected of containing, motile cells.

In one embodiment, when viewed from the top, the sample port is located on one side of the housing.

In one embodiment, the medium port is located on one side of the housing when viewed from the top.

In one embodiment, the medium port and the valve or slit for pressure regulation are located next to each other, when viewed from the top. If this is the case, a single lid or a combined lid may be used to simultaneously cover the medium port and the valve or slit for pressure regulation

In one embodiment, when viewed from the top, the sample port is located on one side of the housing, optionally on one side of the major axis of the chambers, while the medium port and/or the valve or slit for pressure regulation are located on the opposite site of the housing, optionally on the opposite side of the major axis of the chambers.

In one embodiment, the sample port and the valve or slit for pressure regulation are located next to each other, when viewed from the top. If this is the case, a single lid or a combined lid may be used to simultaneously cover the sample port and the valve or slit for pressure regulation.

In one embodiment, the sample port and/or the medium port comprises a connection interface at the end that is directed to the outside of the device that is compatible with a standardized system for fluid fittings used in syringes, wherein, preferably said standardized system for fluid fittings is a Luer taper connection, even more preferably, a Luer lock fitting or a Luer slip fitting.

In one embodiment, an opening of the sample port and/or the medium port that is oriented to the inside of the respective chamber widens in a conical shape.

This widening of the opening in form of a conical shape allows for a gentle insertion and/or extraction of a cell sample and/or a conditioning medium and/or a conditioning medium containing, or suspected of containing, separated motile cells.

In one preferred embodiment, the sample port and/or the medium port comprise a bottleneck such as a throttle, preferably placed at and/or towards the end that is oriented to the inside of the respective chamber. Preferably, this bottleneck has an hourglass-shaped cross-section. Advantageously, the bottleneck prevents liquid from being inserted into, and/or extracted from the respective chamber too quickly. If the injection is too fast, there is a risk that the native sample will be pushed up through the filter membrane when the lower chamber is filled, or that the medium will be pushed down through the cell-permeable membrane when the upper chamber is filled. This can be prevented by the bottleneck.

In preferred embodiments, the angles at the top end of bottleneck and/or the hourglass-shaped cross-section are in a range of about 30° to about 160°, preferably in a range of about 45° to about 135°, more preferably in a range of about 80° to about 100°, most preferably about 90°.

In preferred embodiments, the angles at the bottom end of the bottleneck and/or the hourglass-shaped cross-section are in a range of about 30° to about 160°, preferably in a range of about 80° to about 150°, more preferably in a range of about 100° to about 140°, most preferably about 120°.

In preferred embodiments, the narrowest point of the bottleneck and/or the hourglass-shaped cross-section has a diameter in a range of about 0.1 mm to about 3 mm, preferably in a range of about 0.5 mm to about 1.5 mm, most preferably of about 1 mm.

In one embodiment, the device is a single use device.

In one embodiment, the device contains imprints on the upper surface of the roof of the upper chamber, such as instructions of the sequence of working steps.

The term "contains, or is suspected of containing, motile cells", as used herein, refers to a sample or medium that either has confirmed motile cells present or is believed to have motile cells based on preliminary evidence or suspicion.

In one embodiment, the upper chamber and/or the lower chamber and/or the base unit and/or the insertion unit is made of all potential, biocompatible plastic materials approved for medical device manufacture, such as polystyrene, polycarbonate, polyethylene terephthalate.

In one preferred embodiment, the device is a mesoscale fluidic device.

The term "mesoscale fluidic device", as used herein, refers to a fluidic device designed to operate at the mesoscale, typically involving dimensions and flow rates between the microscale and macroscale, suitable for handling moderate volumes of fluids. Preferably, mesoscale fluidic devices refer to devices designed to handle volumes of about 1 µL to about 1000 mL.

In one embodiments the term mesoscale fluidic device refers to devices designed to handle volumes of about 1 µL to about 100 mL, more preferably volumes of about 1 µL to about 50 mL, even more preferably volumes of about 10 µL to 10 mL.

In one embodiment, the valve or slit for pressure regulation in the upper chamber prevents the cell-permeable membrane from deforming, such as bending, expanding and/or bulging, when the cell sample is inserted into, and/or extracted from, the lower chamber and/or when the conditioning medium is inserted into, and/or extracted from, the upper chamber.

In one aspect, the present invention relates to the use of the device according to the present invention for the separation of motile cells from other components of a cell sample, wherein the other components of the cell sample are selected from one or more of the group comprising non-motile cells, plasma components such as seminal plasma components, hormones, lipids, lipid compounds, dead cells, erythrocytes, leukocytes, precursor cells, other unwanted cells, proteins, enzymes, ions, minerals, sugars, vitamins, antioxidants, pro-inflammatory and anti-inflammatory molecules, nucleotides, nucleosides, contaminating microorganisms, peptides, glycoproteins, metabolites, antimicrobial agents, growth factors, prostanoids, neurotransmitters, extracellular vesicles, nucleic acids, other small molecules, and/or cellular debris;
preferably for the separation of motile sperm cells from other components selected from one or more of the group comprising non-motile sperm cells, seminal plasma components, sperm precursor cells, dead cells, unwanted cells, hormones, proteins and enzymes, ions and minerals, sugars, lipids, vitamins, other organic compounds, antioxidants, pro-inflammatory and anti-inflammatory molecules, nucleotides and nucleosides, contaminating microorganisms, erythrocytes, leukocytes, peptides, glycoproteins, metabolites, antimicrobial agents, growth factors, prostanoids, neurotransmitters, extracellular vesicles, nucleic acids, other small molecules, and/or cellular debris;
more preferably, for the separation of motile sperm cells from non-motile sperm cells, dead cells, seminal plasma components, sperm precursor cells, prostaglandins, and/or proteins.

In one aspect, the present invention relates to the use of the device according to the present invention for the selection and/or enrichment of motile cells from a cell sample, preferably for the selection and/or enrichment of motile sperm cells from a sperm cell sample.

The term "selection and/or enrichment of motile cells from a cell sample", as used herein, refers to the process of isolating and increasing the concentration of motile cells within a sample, separating them from non-motile cells and other components.

In another aspect, the present invention relates to a method for separating motile cells from other components of a cell sample using the device according to the present invention, the method comprising the steps of
1) acquiring a cell sample which contains, or is suspected of containing, motile cells;
2) optionally, adjusting the volume of the cell sample with a sample buffer to a volume that equals at least the volume of the lower chamber, preferably to a volume that is about 5% to about 10% larger than the volume of the lower chamber, thereby resulting in an adjusted cell sample;
3) inserting the cell sample or the adjusted cell sample into the lower chamber via the sample port using a syringe in a volume sufficient to completely fill the lower chamber;
4) inserting a conditioning medium into the upper chamber via the medium port using a syringe in a volume sufficient to completely fill the upper chamber;
5) optionally, placing the one or more removable lids on the sample port, the medium port and/or the valve or slit;
6) incubating the device at a temperature in a range of about 20°C to about 40°C, preferably in a range of about 25°C to about 38°C, more preferably in a range of about 30°C to about 37°C, even more preferably in a range of about 35°C to about 37°C, most preferably at about 37°C, for a time period in a range of about 10 minutes to about 70 minutes; preferably in a range of about 15 minutes to about 60 minutes, more preferably in a range of about 30 minutes to about 45 minutes, wherein during incubation of the device, motile cells move upwards from the lower chamber containing the cell sample or adjusted cell sample through the cell-permeable membrane into the upper chamber containing the conditioning medium;
7) optionally, removing the one or more removable lids; and
8) extracting conditioning medium, preferably a conditioning medium containing, or suspected of containing, motile cells, from the upper chamber via the medium port using a syringe, wherein, preferably, a part of the volume of the conditioning medium is left in the upper chamber, wherein, more preferably about 60% to about 98% of the volume of the conditioning medium in the upper chamber is extracted, even more preferably about 65% to about 90% of the volume, even more preferably about 70% to about 80% of the volume; wherein the motile cells and/or the cell sample are as defined herein.

The term "acquiring a cell sample", as used herein, refers to the process of obtaining a sample containing cells from a biological source for analysis, processing, or experimentation.

One advantage of not completely removing the conditioning medium, preferably a conditioning medium containing, or suspected of containing, motile cells, is that air bubbles, and thereby physical stress for the separated motile cells, are avoided.

In one embodiment, incubating in step 6) is performed using a heating gradient; preferably wherein a temperature in the lower chamber is held lower than a temperature in the upper chamber.

In one preferred embodiment, the temperature in the lower chamber is held at a temperature in a range of about 30°C to about 37°C, preferably in a range of about 32°C to about 36°C, most preferably at about 35°C.

In one preferred embodiment, the temperature in the upper chamber is held at a temperature in a range of about 35°C to about 40°C, preferably in a range of about 36°C to about 38°C, most preferably at about 37°C.

Preferably, said heating gradient enhances the separation of motile cells from other components in a cell sample, preferably the separation of motile sperm cells from other components. Without wishing to be bound to any theory, sperm cells are able to detect and respond to subtle temperature gradients, resulting in directed movement toward warmer regions, a phenomenon termed thermotaxis. Sperm cells exhibit sensitivity to temperature differences within a specific gradient range (approximately 0.014-0.5°C/mm). By applying a controlled thermal gradient, it is possible to influence the directional migration of sperm cells toward a desired location, e.g., towards or through a cell-permeable membrane.

The device according to the present invention enables the use of a heating gradient. In particular, embodiments in which the volume of the upper chamber is lower than the one on the lower chamber, are beneficial for the use of a heating gradient. Moreover, the one or more removable lids prevent evaporation from the upper and/or the lower chamber. This is in particular beneficial if small volumes in the upper and/or the lower chamber, preferably in the upper chamber, are used.

In one embodiment, the method further comprises the step of
9) storing the extracted conditioning medium until use.

In one embodiment, the method further comprises the step of
10) usage of separated motile cells in the extracted conditioning medium, preferably the motile sperm cells, for subsequent diagnostics and/or for assisted reproductive technologies.

In one embodiment, the method further comprises the steps of
11) extracting the incubated cell sample or the incubated adjusted cell sample from the lower chamber via the sample port using a syringe, wherein, preferably, a part of the volume of the incubated cell sample or the incubated adjusted cell sample is left in the upper chamber, wherein, more preferably about 60% to about 98% of the volume of the incubated cell sample or the incubated adjusted cell sample in the lower chamber is extracted, even more preferably about 65% to about 90% of the volume, even more preferably about 70% to about 80% of the volume;
12) storing the extracted incubated cell sample or the incubated adjusted cell sample until use; and/or
13) direct usage of the incubated cell sample or the incubated adjusted cell sample for analysis.

In one embodiment, said further assisted reproductive technologies are selected from the group comprising intra-uterine insemination (IUI), *in-vitro* co-culture of sperm and oocytes for fertilization (conventional *in vitro* fertilization (IVF)), intracytoplasmic sperm injection (ICSI), cryopreservation and cryo-storage procedures, technologies for further cell separation or cell selection like sperm binding assays, fluorescence activated cell sorting (FACS) and magnetic-activated cell sorting (MACS).

In one embodiment, said subsequent diagnostics are selected from the group comprising classification of concentration, motility and/or morphology / morphometry of sperm in the extracted conditioning medium, evaluation of sperm DNA fragmentation, evaluation of DNA condensation, numerical chromosome analysis and functional examinations for viability / membrane integrity, acrosome reaction, capacitation and/or mitochondrial activity.

In one aspect, the present invention relates to a heating system for incubating a device according to present invention, comprising a heating block with a heated lid, wherein the heating system comprises a space in which the device can be placed, so that when the heating system is closed, the heating block is below the lower chamber of the device and the heated lid is on top of the upper chamber of the device;
wherein the heating system further comprises a computer processor, a computer readable storage medium, and a user interface;
wherein the temperature of the heating block and the heated lid can be controlled separately;
wherein, preferably, the heated lid is in direct contact with the essentially planar upper surface of the roof of the upper chamber and/or the heating block is in direct contact with the essentially planar bottom surface of the floor plate of the lower chamber.

In one embodiment, the heating system, preferably the space in the heating system in which the device can be placed, is adapted, preferably structurally adapted, to the shape of the device.

In one preferred embodiment, the space of the heating system including the heated lid has a fitted shape that matches the shape of the device (optionally, including the base unit and the insertion unit), so that the heating block is in direct contact with essentially the entire bottom surface of the floor plate of the lower chamber and/or the heated lid is in direct contact with the entire upper surface of the roof of the upper chamber, when the heating system is closed.

In one preferred embodiment, when the heating system is closed, the heating block is in contact with about 90% of the bottom surface of the floor plate, preferably with about 92% of the bottom surface of the floor plate, more preferably with about 95% of the bottom surface of the floor plate, even more preferably with about 97% of the bottom surface of the floor plate, even more preferably with about 99% of the bottom surface of the floor plate;
and/or
the heated lid is in contact with about 90% of the upper surface of the roof of the upper chamber, preferably with about 92% of the upper surface of the roof of the upper chamber, more preferably with about 95% of the upper surface of the roof of the upper chamber, even more preferably with about 97% of the upper surface of the roof of the upper chamber, even more preferably with about 99% of the upper surface of the roof of the upper chamber.

In one preferred embodiment, the shape of the heated lid is structurally adapted to the shape of the top of the device, in particular the upper chamber including the sample port, the medium port and the valve or slit for pressure regulation, wherein, preferably, the heated lid is structurally adapted to said ports with one or removable lids, optionally a combined removable lid, placed on said sample port, medium port and/or valve or slit.

This fitted shape enables the direct and individual regulation of the temperature within the upper and/or the lower chamber, preferably when applying a temperature gradient, during incubation of the device.

In one embodiment, the heating system has a timer function and/or an alarm function, wherein, preferably, said timer function and/or alarm function is designed to enable easy maintenance of the incubation time and to inform the user when the incubation time has elapsed.

In one embodiment, the device is not part of the heating system.

In one embodiment, the device is part of the heating system.

In one embodiment, the heating system allows for a placement of the device within the fitted shape of the heating system either permanently or over a longer time period, for example, over the whole period of use and incubation. Advantageously, this eliminates the need to move or carry the device during use. Preferably, this significantly reduces any forces, such as rocking, shaking and/or vibrating, which may promote the migration of undesired cells or components from the lower chamber through the cell-permeable membrane into the upper chamber of the device. Thereby the separation capacity of the device and/or the purity of the motile cell fraction in the conditioning medium of the upper chamber is enhanced.

In one embodiment, the heating system further comprises one or more mounts or recesses, preferably one or more heated mounts or recesses, designed to accommodate tubes, such as sample tubes;
wherein preferably, the one or more mounts or recesses are designed to accommodate samples tubes with a volume in a range of about 0.5 to 50 mL. Further preferably, the one or more mounts or recesses, preferably one or more heated mounts or recesses are suitable for clear and defined placement and/or tempering of the tubes and their contents.

For example, the one or more mounts or recesses can be used for defined placement and/or tempering of tubes containing extracted conditioning medium, containing or suspected of containing motile cells prior to the use in subsequent diagnostics and/or further assisted reproductive technologies. The clear assignment of the corresponding tubes to a distinct selection device allows for a defined, standardized workflow and reduces the risk of mixing up samples from different patients. Moreover, the one or more mounts or recesses can be used for prewarming of tubes and their contents, such as sample medium or conditioning medium.

In one embodiment, the computer processor of the heating system can be programmed to run an incubation protocol, wherein, preferably the heated lid and the heating block of the heating system can be controlled individually.

The term "computer processor", as used herein, refers to an electronic circuit or microchip that executes instructions and performs calculations to process data in a system.

In one embodiment, the computer-readable storage medium is designed to store one or more pre-installed and/or manually installed incubation protocols.

The term "computer-readable storage medium", as used herein, refers to any physical device or material that can store data and instructions readable by a computer, including hard drives, solid-state drives, optical discs, and memory cards.

The term "user interface", as used herein, refers to the means by which a user interacts with a system, such as a heating system according to the present invention, including graphical displays, input devices, and software elements that facilitate user commands and feedback.

In one embodiment, the incubation protocol comprises different temperatures for the heating block and the heated lid.

In one embodiment, the incubation protocol comprises information about one or more temperatures of the heating block and/or the heated lid and durations of said one or more temperatures.

In one embodiment, the incubation protocol comprises the information to trigger an alarm sound, e.g. in the event that the end of the incubation time is reached.

In one aspect, the present invention relates to the use of a heating system according to the present invention for separating motile cells from other components of a cell sample, wherein, preferably, the heating system is used in a method according to the present invention;
wherein, further preferably, during incubation of the device in step 6), the temperature of the heating block is set to a lower temperature than the temperature of the heated lid,
wherein, more preferably, the temperature of the heating block is set to a temperature in a range of about 30°C to about 37°C, preferably in a range of about 32°C to about 36°C, most preferably to about 35°C; and the temperature of the heated lid is set to a temperature in a range of about 35°C to about 40°C, preferably in a range of about 36°C to about 38°C, most preferably to about 37°C.

In one embodiment, the device is located and/or positioned in the fitted shape of the heating system prior or during one or more, optionally all, of steps 1) to 10) of the method according to the present invention. It is possible to handle all steps of the method according to the present invention while the device is located in the heating system. Advantageously, this eliminates the need to move or carry the device during use. Preferably, this significantly reduces any forces, such as rocking, shaking and/or vibrating, which may promote the migration of undesired cells or components from the lower chamber through the membrane into the upper chamber of the device. Thereby the separation capacity of the device and/or the purity of the sperm fraction in the conditioning medium of the upper chamber is enhanced.

In one aspect, the present invention relates to a kit for the separation of motile cells from other components of a cell sample, the kit comprising
a) a device according to the present invention; and
one or more of, optionally all of:
b) one or more syringes for inserting a cell sample into or extracting a cell sample from the lower chamber;
c) one or more syringes for inserting conditioning medium into and/or extracting conditioning medium from the upper chamber;
d) a sample buffer;
e) a conditioning medium;
f) a sterile collection container for the cell sample;
g) one or more sterile capillaries for aspirating and/or dispensing the cell sample and/or the conditioning medium into the corresponding syringes of b) and/or c); or
h) a heating system according to the present invention.

In a preferred embodiment, the components of the kit are sterile and/or packed under sterile conditions.

### BRIEF DESCRIPTION OF THE FIGURES

The present invention is now further described by reference to the following figures.

All methods mentioned in the figure descriptions below were carried out as described in detail in the examples. Moreover, exemplary devices displayed in the figure and described figure captions below are given to illustrate, not to limit the present invention.
- **Figure 1:**: **Sectional view of an exemplary device according to the present invention.**
- **Figure 2:**: **Exemplary insertion units without base unit as seen from the bottom.**
- **Figure 3:**: **Exemplary insertion units without base unit as seen from the top.**
- **Figure 4:**: **Exemplary base units without insertion unit as seen from the top.**
- **Figure 5:**: **Exemplary base units without insertion unit as seen from the bottom.**
- **Figure 6:**: **Assembled exemplary device of the present invention without lid.**
- **Figure 7:**: **Assembled exemplary device of the present invention with a combined lid.**
- **Figure 8:**: **Example of a combined lid.**
- **Figure 9:**: **Exploded view of exemplary devices according to the present invention.**
- **Figure 10:**: **Use of a device according to the present invention.**
- **Figure 11:**: **Analysis of Zinc (Zn²⁺) concentration in the upper chamber after incubation as a marker for the retention capacity of a filter membrane.**

In the following, reference is made to the examples, which are given to illustrate, not to limit the present invention.

### EXAMPLES

### Example 1

### Detailed description of exemplary devices

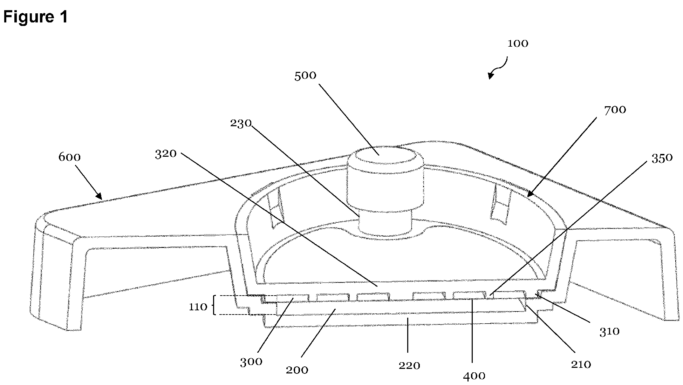

**Figure 1-9** display possible embodiments of devices of the present invention.

**Figure 1** shows a sectional view of an exemplary device **100** according to the present invention. In this embodiment, the side walls **210** and the floor plate **220** of the lower chamber **200** are part of a base unit **600.** The outer walls of the base unit **600** starting from the lower end to the upper end that are angled inwards are displayed at the outermost part of the device **100.** The inwards angle can help to enhance the stability of the device **100.** At the upper end of the outer walls, a horizontal platform is displayed which contains a groove. This groove may have the same general shape as the lower chamber **200** when viewed from top. The groove has inner walls which extend from the horizontal platform downwards, wherein the lower end of the inner walls of the base unit **600** are connected to the upper end of the side walls of the lower chamber **210.** As displayed in the exemplary device **100,** in some embodiments, the inner walls of the base unit **600** within the groove comprise a horizontal inwards protruding ring at the bottom, and the side walls of the lower chamber **210** comprise a horizontal outwards protruding ring at the top. The inwards protruding ring of the inner walls of the base unit **600** is directly connected to the outwards protruding ring of the side walls of the lower chamber **210.** The inner walls of the base unit **600** within said groove starting from the lower end to the upper end may be angled outwards.

Concerning the upper chamber **300,** the side walls **310,** the inner walls **350,** and the roof **320** may be part of an insertion unit **700.** Said insertion unit **700** may comprise outer walls located at the top of the roof of the upper chamber **320.** Said insertion unit **700,** in particular said outer walls of the insertion unit **700,** are structurally adapted to sit within the groove of the horizontal platform of the base unit **600.** In the exemplary device **100** said insertion unit **700** is inserted into the groove of the base unit **600,** so that the lower chamber **200** and the upper chamber **300** are assembled in the housing **110.** The side walls of the upper **310** and the side walls of the lower chamber **210** are aligned with each other so that the housing **110** is closed, apart from the sample port **230** of the lower chamber **200,** the medium port **330** (not shown) and the valve or slit for pressure regulation **340** (not shown).

The upper chamber **300** and the lower chamber **200** may be in an essentially oval shape when viewed from the top. The cell-permeable membrane **400** is located between the lower chamber **200** and the upper chamber **300,** when the lower chamber **200** and the upper chamber **300** are assembled in the housing **110.** In the sectional view of the device **100,** within the area between the side walls of the upper chamber **310,** different sections of the continuous circuitous flow path of the upper chamber **300** are displayed in **Figure 1****.** In one embodiment, this continuous circuitous flow path of the upper chamber **300** is formed by the side walls of the upper chamber **310** and one or more inner walls within the upper chamber **350.**

As can be seen in **Figure 1****,** the sample port **230** may run through the insertion unit **700** and/or the upper chamber **300,** to connect the lower chamber **200** with the outside at the top of the device **100.** In one embodiment, when not in use, the sample port **230** may be covered by a removable lid **500** to prevent contamination and/or evaporation. This removable lid **500** can be in form of a single lid.

In some embodiments, the inner walls and/or the horizontal platform of the base unit **600** can have one or more recesses (such as holes, mortises, or slots). The outer walls of the insertion unit **700** may have one or more protrusions at the corresponding positions (such as "latching noses", pins, tenons, or lugs), so that when said insertion unit **700** is inserted into the groove of the base unit **600,** the connection between the insertion unit **700** and the base unit **600** is stabilized.

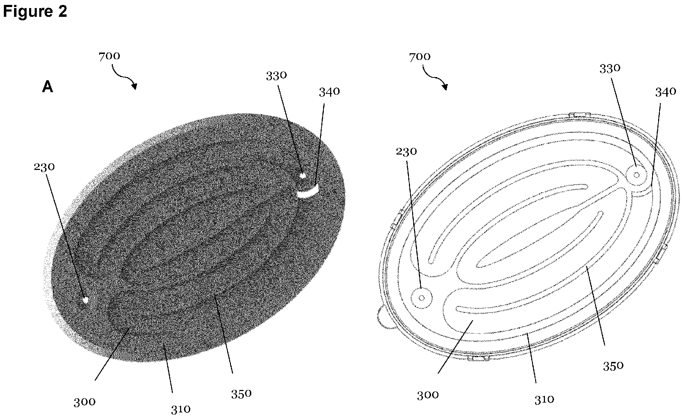

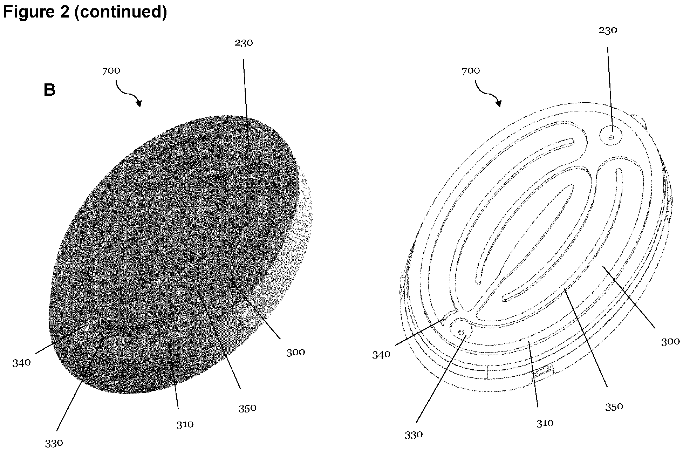

**Figure 2** shows exemplary embodiments of an insertion unit **700,** that may be used, for example, in one of the exemplary devices **100** shown in Figure 1. **Figure 2A** displays a bottom view of an exemplary insertion unit **700** with upper chamber **300** without protrusions at the outer walls (left; 3D representation) or with protrusions at the outer walls (right; technical drawing). **Figure 2B** shows a bottom view of the insertion unit **700** with upper chamber **300** in an angled view without protrusions at the outer walls (left; 3D representation) or with protrusions at the outer walls (right; technical drawing). The upper chamber **300** in form of a continuous circuitous flow path is displayed. In some embodiments, the continuous circuitous flow path is formed by the side walls of the upper chamber **310** and one or more inner walls within the upper chamber **350.** For example, the continuous circuitous flow path may be formed by the side walls of the upper chamber **310** and five inner walls **350,** one of which may be a central inner wall with 2 parallel inner walls of the upper chamber on each side.

On one end of the continuous circuitous flow path, the roof of the upper chamber **320** comprises a medium port **330** that connects the upper chamber **300** to the outside. At the other end of the continuous circuitous flow path, the roof **320** comprises a valve or slit **340** that connects the upper chamber **300** to the outside for pressure regulation.

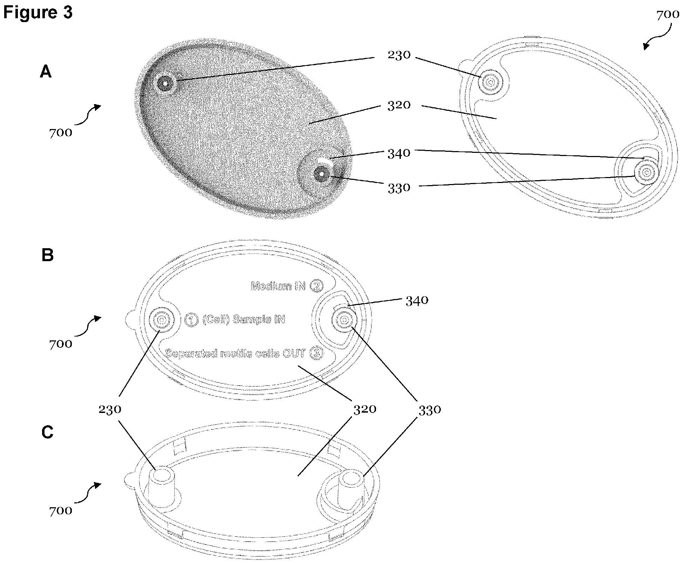

In **Figure 3** exemplary embodiments of an insertion unit **700,** that may be used, for example, in one of the exemplary devices **100** shown in Figure 1, are displayed as seen from the top. **Figure 3A** displays a top view of exemplary insertion units **700** without protrusions at the outer walls (left; 3D representation) or with protrusions at the outer walls (right; technical drawing). In some embodiments, the insertion unit **700** can comprise a labeling of the handling steps (cf. for example **Figure 3B**). In **Figure 3C** an angled view on top of a possible insertion unit **700** is displayed.

The groove of the insertion unit **700** and/or the upper chamber **300** may be in an essentially oval shape when viewed from the top. In some embodiments, the sample port **230** is located on one side of the major axis of the chambers. In some embodiments, the medium port **330** and/or the valve or slit for pressure regulation **340** are located on the opposite side of the major axis of the chambers.

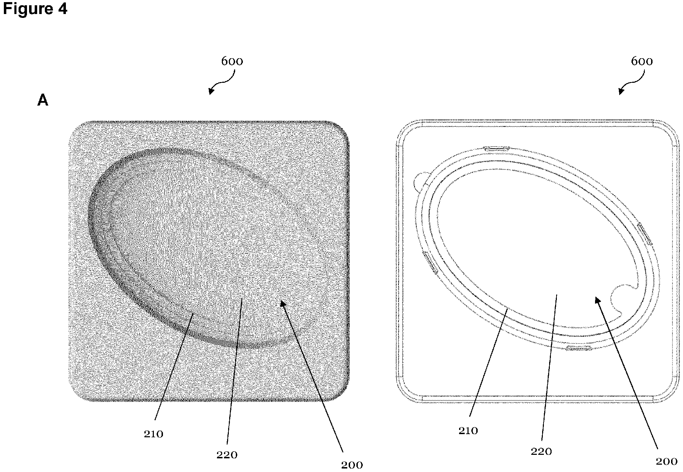

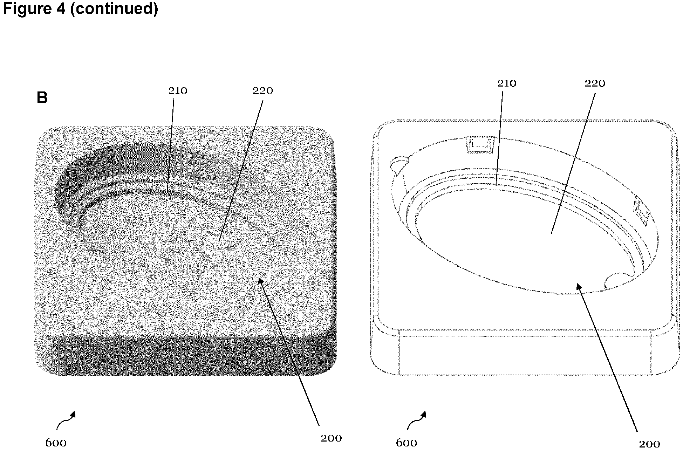

**Figure 4** displays possible embodiments of a base unit **600** that may be used, for example, in one of the exemplary devices **100** shown in Figure 1. **Figure 4A** shows a top view of a possible base unit **600** without recesses (left; 3D representation), or, optionally, with one or more recesses in the inner walls of the base unit **600** (right; technical drawing). **Figure 4B** shows a top view of a possible base unit **600** viewed from an angle without recesses (left; 3D representation) or with recesses (right; technical drawing). The lower chamber **200** with its side walls **210** and the floor plate **220** may be located in a central part of the base unit **600.**

In some embodiments, protrusions at the corresponding positions of the outer walls of an insertion unit **700** (not shown) can fit into the one or more recesses in the inner walls of the base unit **600,** so that when said insertion unit **700** is inserted into the groove of the base unit **600,** the connection between the insertion unit **700** and the base unit **600** is stabilized.

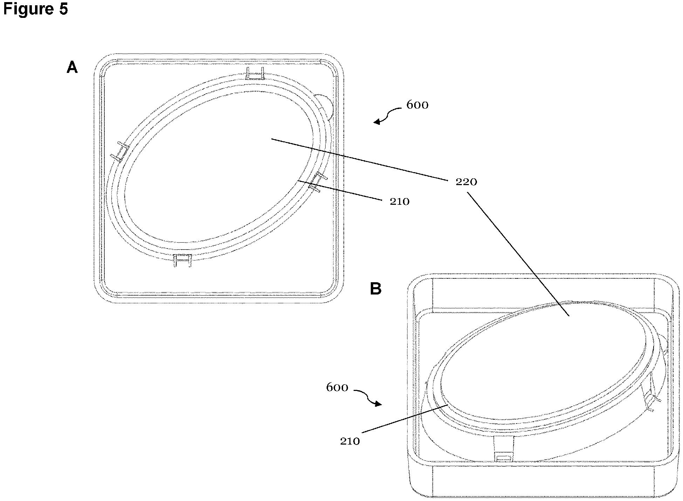

**Figure 5** displays possible embodiments of a base unit **600,** viewed from the bottom, that may be used, for example, in one of the exemplary devices **100** shown in Figure 1. **Figure 5A** displays a bottom view of a possible base unit **600.** In **Figure 5B** an angled view at the bottom of said base unit **600** is shown. The inner walls of the base unit **600** may have one or more recesses in which protrusions at the corresponding positions of the outer walls of an insertion unit **700** fit, so that when said insertion unit **700** is inserted into the groove of the base unit, the connection between the insertion unit **700** and the base unit **600** is stabilized.

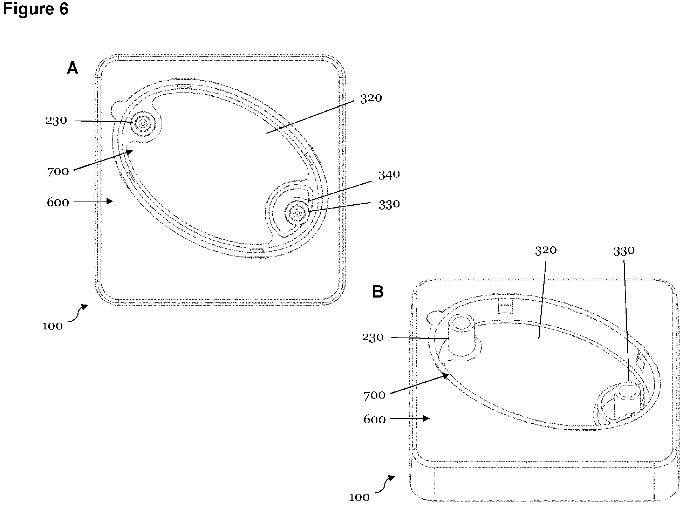

**Figure 6** displays an exemplary device **100** according to the present invention in an assembled form (**Figure 6A**: Top view of a possible assembled device **100;** **Figure 6B****:** Angled view at the top of a possible assembled device **100**). The groove of the insertion unit **700** and/or the upper chamber **300,** that is confined to the top by the roof **320,** may be in an essentially oval shape when viewed from the top. In some embodiments, the sample port **230** is located on one side of the major axis of the chambers. In some embodiments, the medium port **330** and/or the valve or slit for pressure regulation **340** are located on the opposite side of the major axis of the chambers. The medium port **330** and/or the valve or slit for pressure regulation **340** can be arranged side by side.

The outer walls of the insertion unit 700 may have one or more protrusions (such as "latching noses", pins, tenons, or lugs) that fit into one or more recesses (such as holes, mortises, or slots) of a base unit 600 at the corresponding positions.

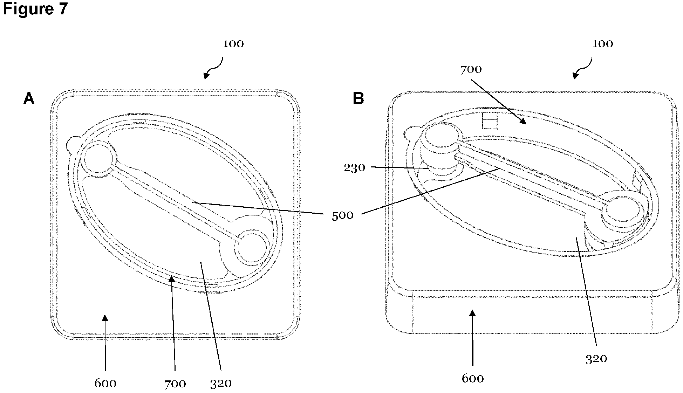

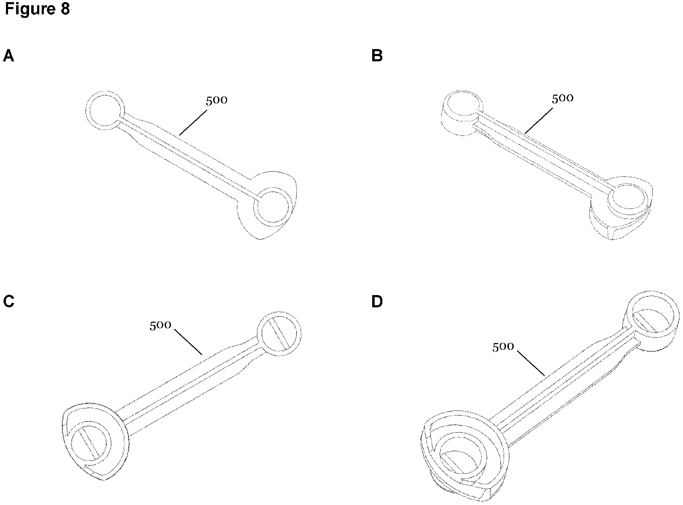

Figure **7** shows an embodiment of the present invention which is comparable to the one in Figure 6. In addition to the embodiment of Figure 6, Figure **7** displays a removable lid 500 in form of a combined lid that can be simultaneously placed on or removed from at least two of the sample port 230 of the lower chamber 200, the medium port 330 of the upper chamber 300, and/or the valve or slit 340 of the upper chamber 300. A detailed view of a possible embodiments of a removeable lid 500 in form of combined lids that may be used, for example, in the embodiments of Figures **1-7****,** are displayed in Figure 8A-D. Figure 8A displays a top view of a possible combined lid. **Figure 8B** shows a top view of a possible combined lid viewed from an 45° angle. **Figure 8C** shows a bottom view of a possible combined lid and **Figure 8D** a bottom view of viewed from an 45° angle.

Advantageously, a combined lid enables the simultaneous covering of at least two of the sample port **230** of the lower chamber **200,** the medium port **330** of the upper chamber **300,** and/or the valve or slit **340** of the upper chamber **300.** This simplifies and speeds up the handling of the lid **500** and reduces the number of parts, in particular small parts like individual lids. Moreover, the combined lid has haptic advantages as it is easier to be grabbed by a person working with a device **100** of the present invention. Also, the reduced number of parts reduces the risk of mismatching lids to the wrong devices in case several devices are used at the same time.

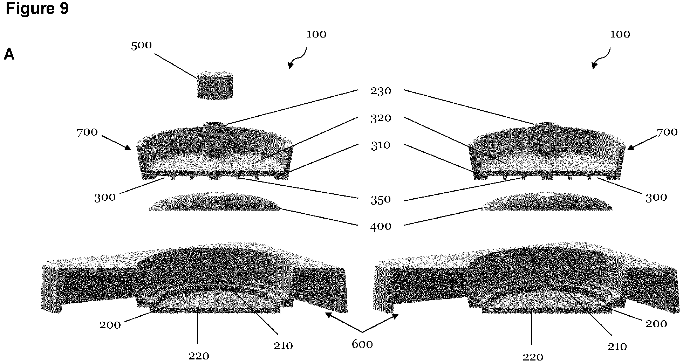

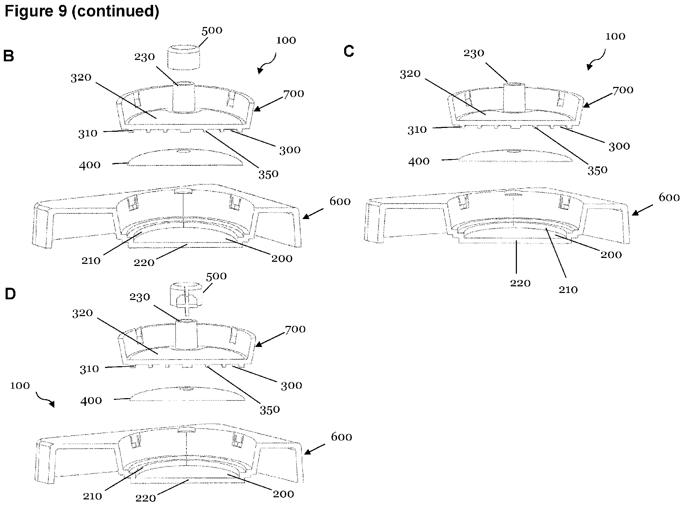

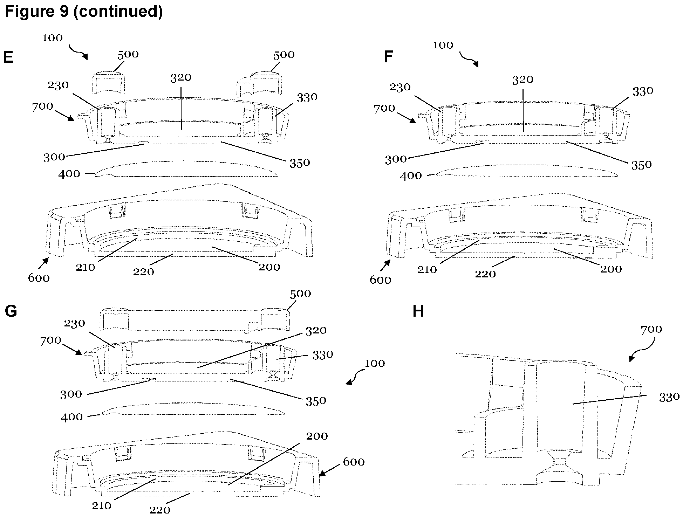

**Figure 9** displays exploded views of embodiments that are comparable to the ones of **Figure 1****.** The cell-permeable membrane **400** is located between lower chamber **200** and the upper chamber **300.** The exploded views shown in **Figures 9A-D** display embodiments of possible devices **100** that are cut along the minor axis of the chambers. In some embodiments, the device **100** can be present without lid **(****Figures 9A** right panel, **Figure 9C****).** In some embodiments, a lid **500** in form of a single lid may be placed on the sample port **230,** the medium port **330** and/or the valve or slit **340 (****Figure 9A** left panel, **Figure 9B****).** In some embodiments, a lid **500** in form of a combined lid may be placed on the sample port **230,** the medium port **330** and/or the valve or slit **340 (****Figure 9D****).**

The exploded views shown in **Figures 9E-G** display embodiments of possible devices **100** that are cut along the major axis of the chambers. In some embodiments, the device **100** can be present without lid **(****Figures 9F****).** In some embodiments, a lid **500** in form of a single lid may be placed on the sample port **230** and/or a lid **500** in form of a combined lid may be simultaneously placed on the medium port **330** and the valve or slit for pressure regulation **340 (****Figure 9E****).** In some embodiments, a lid **500** in form of a combined lid may be placed on the sample port **230,** the medium port **330** and the valve or slit **340 (****Figure 9G****).**

In some embodiments, the sample port **230** and/or the medium port **330** may comprise a bottleneck such as a throttle, preferably placed at the end that is oriented to the inside of the respective chamber **(****Figure 9H****).** Preferably, this bottleneck has an hourglass-shaped cross-section. Advantageously, the bottleneck prevents liquid from being inserted into, and/or extracted from the respective chamber too quickly.

### Example 2

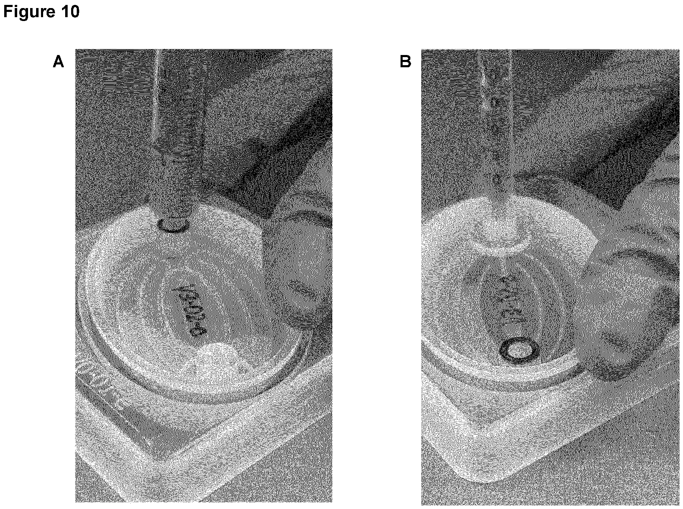

The handling of an exemplary device according to the present invention is shown in **Figure 10****.** For an easier identification of the ports, the sample port has been marked with a black circle. In a first step, the lower chamber is filled via the sample port using a syringe **(****Figure 10A). Figure 10B** displays the introduction of a solution into the upper chamber in a subsequent step. During filling of the upper chamber, the liquid follows the continuous circuitous flow path starting from the sample port in direction of the medium port. As can be seen from **Figure 10B****,** the form of the flow path enables a gentle insertion of the liquid with no occurrence of air-bubbles. Moreover, during filling of each chamber, the membrane maintained its form, due to the stabilizing and supporting effects of the continuous circuitous flow path in the upper chamber.

### Example 3

### Passive diffusion of zinc ions through a filter membrane as a marker for filter retention capacity

### Materials

Test containers: Cylindric prototype device for selection of motile cells made of aluminum divided into a lower chamber and an upper chamber. The cell-permeable membrane is mounted horizontally between the two chambers.

Sample tubes: Eppendorf tubes, 2 ml.

Test membrane for cell-permeable membrane: track-etched membrane, PET, thickness 17 µm, pore diameter 7 µm, open area 7-10 %.

Sperm preparation medium: Fujifilm Irvine Scientific Multipurpose Handling Medium-Complete (MHM-C) with Gentamicin.

Cell sample: Native ejaculate from three voluntary donors.

### Method

The lower chamber of the device was filled with 2 mL of liquefied, native ejaculate. The upper chamber was filled with 1.5 mL of pre-warmed sperm preparation medium (conditioning medium). After filling, the devices were incubated in a humidified incubator at 37°C for 30 min to allow for sperm from the lower chamber to migrate through the filter membrane into the medium of the upper chamber and for non-sperm components of native ejaculate to passively diffuse into the upper chamber. The test was run in 5 duplicates.

After 30 min, 1 mL of medium was gently withdrawn from the upper chamber of each device and transferred into Eppendorf tubes. Sperm concentration and motility was determined from 3 µL aliquots of the five samples.

Reduction in the concentration of the unwanted components in the upper chamber of the device after incubation was exemplary assessed via determination of zinc (Zn²⁺) concentration in the conditioning medium after incubation. Zinc ions are very small and can be used as a marker for the retention capacity of a membrane. Moreover, zinc analysis is commonly carried out to determine prostate function.

For the analysis of the zinc concentration, the remaining volumes of the extracted conditioning media were frozen for further analysis. For control experiments, corresponding 1 mL aliquots of pure sperm preparation medium (negative control, 2 duplicates) and native ejaculate (positive control, 3 duplicates) were also frozen for further analysis.

Frozen samples (10 in total) were shipped to and analyzed by Synlab Labor München Zentrum MVZ GbR for zinc, Zn²⁺.

### Results and discussion

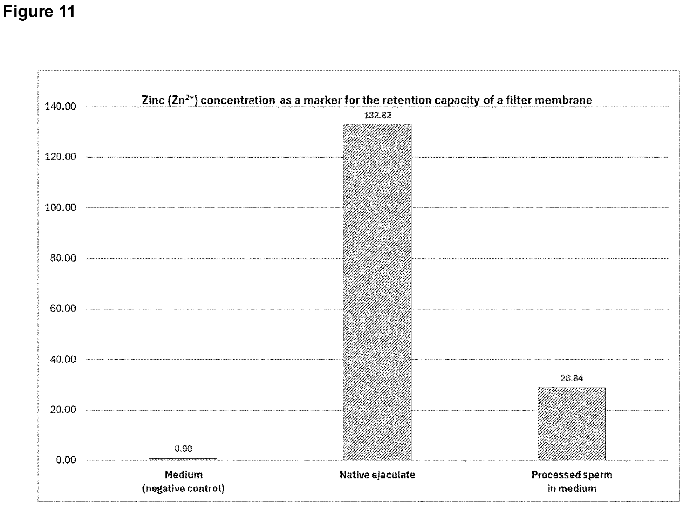

Mean zinc concentrations were 0.9 µg/mL in the negative control, 132.82 µg/mL in native ejaculate, and 28.84 µg/mL in the five test samples (sperm preparation medium from the upper chamber) **(****Figure 11****).** The dilution coefficient (mean zinc concentration in the test samples - base level in the negative control / native zinc concentration in ejaculate) was 0.21.

Despite its ultra-low thickness and its high open area, the tested filter membrane was able to efficiently retain a very small marker molecule like Zn²⁺ over an incubation period of 30 min and significantly reduce passive diffusion from native ejaculate into a fraction of clean culture medium.

The results show that the filtration and purification process over the membrane is effective with respect to deselecting even smallest components of seminal plasma while motile sperm are able to pass the membrane.

### Example 4

### Conclusion

The results of the experiments show that the filtration and purification process over the membrane is effective with respect to deselecting even smallest components of seminal plasma while motile sperm are able to pass the membrane. Moreover, the continuous circuitous flow path of the upper chamber enabled a gentle, air-bubble free filling of the upper chamber, and the membrane maintained its form, due to the stabilizing and supporting effects of the continuous circuitous flow path in the upper chamber.

This demonstrates that the device according to the present invention is highly suitable for an improved separation of motile cells from other components of a cell sample. Advantageously, the device addresses the issues of uneven pore distribution and membrane deformation. By this, the device according to the present invention does not face scalability limitations since larger volumes of the lower chamber and/or the upper chamber can be used. Moreover, the stabilization of the membrane enables devices with one or more further advantages such as a larger cell-permeable membrane, a larger surface area of the cell-permeable membrane, or a higher volume ratio of the lower chamber compared to the upper chamber. Moreover, devices, such as those shown in **Figures 1****,** **8** and **9****,** address the issues of liquid evaporation and contamination by the use of a lid that covers the different ports or the valve. By overcoming these challenges, the device according to the present invention significantly enhances the safety, efficiency and effectiveness of motile cell separation, providing valuable benefits for both research and clinical applications, in particular assisted reproductive technologies. Moreover, the improved device according to the present invention can separate motile cells, not only from non-motile or dead cells but also from other components that would act as disruptive factors in the further use or processing of separated motile cells, which can be seen by the retention of the very small molecule zinc.

### LIST OF REFERENCE SIGNS

- 100: device for separating motile cells
- 110: housing

- 200: lower chamber
- 210: side walls (of the lower chamber)
- 220: floor plate (of the lower chamber)
- 230: sample port

- 300: upper chamber
- 310: side walls (of the upper chamber)
- 320: roof (of the upper chamber)
- 330: medium port
- 340: valve or slit
- 350: inner wall(s) within the upper chamber

- 400: cell-permeable membrane

- 500: removable lid

- 600: base unit

- 700: insertion unit

## Claims

1. A device (100) for separating motile cells from other components of a cell sample, the device (100) comprising a housing (110), which comprises:
1) a lower chamber (200) which is open at the top and confined to the sides by side walls (210) and to the bottom by a floor plate (220),
wherein a bottom surface of the floor plate (220) facing the outside is essentially planar,
wherein a sample port (230) connects the lower chamber (200) to the outside,
wherein the lower chamber (200) is designed to accommodate a cell sample which may be inserted into, and/or extracted from, the lower chamber (200) via the sample port (230), wherein the cell sample contains, or is suspected of containing, motile cells;
2) an upper chamber (300) which is open at the bottom and confined to the sides by side walls (310) and to the top by a roof (320),
wherein an upper surface of the roof (320) facing the outside is essentially planar,
wherein within the area between the side walls (310), the upper chamber (300) is arranged in form of a continuous circuitous flow path, preferably in form of a serpentine flow path,
wherein on one end of the continuous circuitous flow path, preferably the serpentine flow path, the roof (320) comprises a medium port (330) that connects the upper chamber (300) to the outside, and at the other end of the continuous circuitous flow path, the roof (320) comprises a valve or slit (340) that connects the upper chamber (300) to the outside for pressure regulation;
wherein the upper chamber (300) is designed to accommodate a conditioning medium which may be introduced into, and/or extracted from, the upper chamber (300) via the medium port (330);
wherein the upper chamber (300) is located on top of the lower chamber (200);
wherein, when the lower chamber (200) and the upper chamber (300) are assembled in the housing (110), the side walls of the upper and lower chamber (310, 210) are aligned with each other so that the housing (110) is closed, apart from the sample port (230) of the lower chamber, the medium port (330) of the upper chamber and the valve or slit (340) for pressure regulation;
3) a cell-permeable membrane (400) which is located between the lower chamber (200) and the upper chamber (300), wherein the cell-permeable membrane (400) is located on top of the lower chamber (200) and below the upper chamber (300);
and optionally
4) one or more removable lids (500), wherein the one or more removable lids (500) are placed on the sample port (230) of the lower chamber, the medium port (330) of the upper chamber, and/or the valve or slit (340) of the upper chamber;
wherein the other components of the cell sample are selected from one or more of the group comprising non-motile cells, plasma components such as seminal plasma components, hormones, lipids, lipid compounds, dead cells, erythrocytes, leukocytes, precursor cells, other unwanted cells, proteins, enzymes, ions, minerals, sugars, vitamins, antioxidants, pro-inflammatory and anti-inflammatory molecules, nucleotides, nucleosides, contaminating microorganisms, bacteria, fungal spores, peptides, glycoproteins, metabolites, antimicrobial agents, growth factors, prostanoids, neurotransmitters, extracellular vesicles, nucleic acids, other small molecules, and cellular debris.

2. The device (100) according to claim 1, wherein the motile cells are selected from motile eukaryotic cells, such as motile mammalian sperm cells or motile avian sperm cells; preferably selected from motile sperm cells from a species of an order selected from the orders of primates, artiodactyla, perissodactyla, carnivora, cetacea, chiroptera, rodentia, accipitriformes, falconiformes, galliformes; more preferably selected from motile human sperm cells, bovine sperm cells, porcine sperm cells, equine sperm cells, caprine sperm cells, canine sperm cells, feline sperm cells, ovine sperm cells, dolphin sperm cells, bat sperm cells, rat sperm cells, hawk sperm cells, falcon sperm cells, and turkey sperm cells; even more preferably motile human sperm cells, bovine sperm cells, porcine sperm cells, and equine sperm cells; most preferably motile human sperm cells.

3. The device (100) according to claim 1 or 2, wherein the cell sample is a mammalian semen sample or an avian semen sample; preferably a semen sample from a species of an order selected from the orders of primates, artiodactyla, perissodactyla, carnivora, cetacea, chiroptera, rodentia, accipitriformes, falconiformes, galliformes; more preferably the cell sample is selected from a human semen sample, bovine semen sample, porcine semen sample, equine semen sample, caprine semen sample, canine semen sample, feline semen sample, ovine semen sample, dolphin semen sample, bat semen sample, rat semen sample, hawk semen sample, falcon semen sample, turkey semen sample; more preferably human semen sample, bovine semen sample, porcine semen sample, equine semen sample; most preferably a human semen sample;
wherein, further preferably, the seminal fluid is a native seminal fluid.

4. The device (100) according to any of the foregoing claims, wherein the other components of the cell sample are selected from one or more of the group comprising non-motile sperm cells, seminal plasma components, sperm precursor cells, dead cells, unwanted cells, hormones, proteins and enzymes, ions, minerals, sugars, lipids, vitamins, other organic compounds, antioxidants, pro-inflammatory and anti-inflammatory molecules, nucleotides and nucleosides, contaminating microorganisms, bacteria, fungal spores, peptides, glycoproteins, metabolites, antimicrobial agents, growth factors, prostanoids, neurotransmitters, extracellular vesicles, nucleic acids, other small molecules, and cellular debris;
wherein, preferably, the other components are selected from one or more of the group comprising non-motile sperm cells, dead cells, seminal plasma components, sperm precursor cells, unwanted cells, hormones, lipids, proteins and enzymes, contaminating microorganisms, bacteria, and fungal spores;
even more preferably selected from non-motile sperm cells, dead cells, seminal plasma components, contaminating microorganisms, bacteria, and fungal spores;
most preferably non-motile sperm cells, dead cells, seminal plasma components.

5. The device (100) according to any of the foregoing claims, wherein the continuous circuitous flow path, preferably the serpentine flow path, of the upper chamber (300) is formed by the side walls of the upper chamber (310) and one or more inner walls within the upper chamber (350);
and/or
wherein the upper chamber (300) in form of the continuous circuitous flow path, such as the serpentine flow path, allows for a gentle insertion and/or extraction of a conditioning medium, wherein, preferably, the insertion and/or extraction of the conditioning medium is air-bubble free.

6. The device (100) according to any of the foregoing claims, wherein the continuous circuitous flow path, such as the serpentine flow path, of the upper chamber (300), preferably the inner walls of said upper chamber (350) forming said continuous circuitous flow path, serves as support for the cell-permeable membrane (400), thereby stabilizing the cell-permeable membrane (400);
wherein, more preferably, said support for the cell-permeable membrane (400) prevents the cell-permeable membrane from deforming, such as bending, expanding and/or bulging, when the cell sample is inserted into, and/or extracted from, the lower chamber (200) and/or when the conditioning medium is inserted into, and/or extracted from, the upper chamber (300); and/or
wherein, further preferably, said support for the cell-permeable membrane (400) enables
larger volumes of the lower chamber (200) and/or the upper chamber (300), and/or
a higher volume ratio of the lower chamber (200) versus the volume of the upper chamber (300), and/or
a larger cell-permeable membrane (400), and/or
a larger surface area of the cell-permeable membrane (400), and/or
a higher ratio of surface area of the cell-permeable membrane (400) to volume of the lower chamber (200) and/or the upper chamber (300),
when compared to a device without an upper chamber in form of a continuous circuitous flow path, preferably a serpentine flow path.

7. The device (100) according to any of the foregoing claims, wherein the volume of the lower chamber (200) is in a range of about 500 µL to about 5 mL, preferably in a range of about 750 µL to about 4 mL, more preferably in a range of about 1 mL to about 3 mL, even more preferably in a range of about 1.5 mL to about 2.5 mL, most preferably the volume of the lower chamber (200) is about 2 mL;
and/or
wherein the volume of the upper chamber (300) is in a range of about 100 µL to about 2 mL, preferably in a range of about 400 µL to about 1.6 mL, more preferably in a range of about 600 µL to about 1.2 mL, even more preferably in a range of about 700 µL to about 1 mL, most preferably the volume of the upper chamber (300) is about 850 µL;
and/or
wherein the volume of the lower chamber (200) is larger than the one of the upper chamber, preferably by a factor in a range of about 1.2 to about 4, more preferably in a range of about 1.5 to about 3.5, even more preferably in a range of about 2 to about 3, even more preferably in a range of about 2 to about 2.5, most preferably by a factor of about 2.35.

8. The device (100) according to any of the foregoing claims, wherein a larger volume of the lower chamber (200) compared to the volume of the upper chamber (300) increases the yield of motile cells, such as motile healthy sperm cells, and thus a greater selection efficiency
when compared to a device in which the volumes of the upper chamber (300) and the lower chamber (200) is about similar, or in which the volume of the upper chamber (300) is larger than the volume of the lower chamber (200),
or when compared to a device in which the volume of the lower chamber (200) is not larger than the one of the upper chamber (300), preferably by a factor in a range of about 1.2 to about 4, more preferably in a range of about 1.5 to about 3.5, even more preferably in a range of about 2 to about 3, even more preferably in a range of about 2 to about 2.5, most preferably by a factor of about 2.35.

9. The device (100) according to any of the foregoing claims, wherein the cell-permeable membrane (400) comprises pores with a pore size in the range of about 5 µm to about 12 µm, preferably in the range of about 5 µm to about 10 µm, more preferably in the range of about 5 µm to about 8 µm, most preferably a pore size of about 7 µm;
and/or
wherein the cell-permeable membrane (400) is a monofilament fabric, preferably a monofilament fabric made of polyamide; or wherein the cell-permeable membrane (400) is a track-etched membrane, preferably a track-etched membrane made of polyester;
and/or
wherein the cell-permeable membrane (400) has an open area in a range of about 0.5% to about 20% of a total surface area of the membrane, preferably in a range of about 0.75% to about 15%, more preferably in a range of about 1% to about 10%.

10. Use of the device (100) according to any of claims 1-9 for the separation of motile cells from other components of a cell sample, wherein the other components of the cell sample are selected from one or more of the group comprising non-motile cells, plasma components such as seminal plasma components, hormones, lipids, lipid compounds, dead cells, erythrocytes, leukocytes, precursor cells, other unwanted cells, proteins, enzymes, ions, minerals, sugars, vitamins, antioxidants, pro-inflammatory and anti-inflammatory molecules, nucleotides,
nucleosides, contaminating microorganisms, peptides, glycoproteins, metabolites, antimicrobial agents, growth factors, prostanoids, neurotransmitters, extracellular vesicles, nucleic acids, other small molecules, and/or cellular debris;
preferably for the separation of motile sperm cells from other components selected from one or more of the group comprising non-motile sperm cells, seminal plasma components, sperm precursor cells, dead cells, unwanted cells, hormones, proteins and enzymes, ions and minerals, sugars, lipids, vitamins, other organic compounds, antioxidants, pro-inflammatory and anti-inflammatory molecules, nucleotides and nucleosides, contaminating microorganisms, erythrocytes, leukocytes, peptides, glycoproteins, metabolites, antimicrobial agents, growth factors, prostanoids, neurotransmitters, extracellular vesicles, nucleic acids, other small molecules, and/or cellular debris;
more preferably, for the separation of motile sperm cells from non-motile sperm cells, dead cells, seminal plasma components, sperm precursor cells, prostaglandins, and/or proteins.

11. Use of the device (100) according to any of claims 1-9 for the selection and/or enrichment of motile cells from a cell sample, preferably for the selection and/or enrichment of motile sperm cells from a sperm cell sample.

12. A method for separating motile cells from other components of a cell sample using the device (100) according to any of claims 1-9, the method comprising the steps of
1) acquiring a cell sample which contains, or is suspected of containing, motile cells;
2) optionally, adjusting the volume of the cell sample with a sample buffer to a volume that equals at least the volume of the lower chamber (200), preferably to a volume that is about 5% to about 10% larger than the volume of the lower chamber (200), thereby resulting in an adjusted cell sample;
3) inserting the cell sample or the adjusted cell sample into the lower chamber (200) via the sample port (230) using a syringe in a volume sufficient to completely fill the lower chamber (200);
4) inserting a conditioning medium into the upper chamber (300) via the medium port (330) using a syringe in a volume sufficient to completely fill the upper chamber (300);
5) optionally, placing the one or more removable lids (500) on the sample port (230), the medium port (330) and/or the valve or slit (340);
6) incubating the device (100) at a temperature in a range of about 20°C to about 40°C, preferably in a range of about 25°C to about 38°C, more preferably in a range of about 30°C to about 37°C, even more preferably in a range of about 35°C to about 37°C, most preferably at about 37°C, for a time period in a range of about 10 minutes to about 70 minutes; preferably in a range of about 15 minutes to about 60 minutes, more preferably in a range of about 30 minutes to about 45 minutes, wherein during incubation of the device, motile cells move upwards from the lower chamber (200) containing the cell sample or adjusted cell sample through the cell-permeable membrane (400) into the upper chamber (300) containing the conditioning medium;
7) optionally, removing the one or more removable lids (500); and
8) extracting conditioning medium, preferably a conditioning medium containing, or suspected of containing, motile cells, from the upper chamber (300) via the medium port (330) using a syringe, wherein, preferably, a part of the volume of the conditioning medium is left in the upper chamber (300), wherein, more preferably about 60% to about 98% of the volume of the conditioning medium in the upper chamber (300) is extracted, even more preferably about 65% to about 90% of the volume, even more preferably about 70% to about 80% of the volume;
wherein the motile cells and/or the cell sample are as defined in any of claims 1-9;
wherein, optionally, incubating in step 6) is performed using a heating gradient; wherein, preferably, a temperature in the lower chamber (200) is held lower than a temperature in the upper chamber (300); wherein, more preferably, the temperature in the lower chamber (200) is held at a temperature in a range of about 30°C to about 37°C, even more preferably in a range of about 32°C to about 36°C, most preferably at about 35°C, and/or wherein the temperature in the upper chamber (300) is held at a temperature in a range of about 35°C to about 40°C, even more preferably in a range of about 36°C to about 38°C, most preferably at about 37°C.

13. A heating system for incubating a device (100) according to any of claims 1-9, comprising a heating block with a heated lid, wherein the heating system comprises a space in which the device (100) can be placed, so that when the heating system is closed, the heating block is below the lower chamber (200) of the device (100) and the heated lid is on top of the upper chamber of the device (100);
wherein the heating system further comprises a computer processor, a computer readable storage medium, and a user interface;
wherein the temperature of the heating block and the heated lid can be controlled separately;
wherein, preferably, the heated lid is in direct contact with the essentially planar upper surface of the roof (320) of the upper chamber (300) and/or the heating block is in direct contact with the essentially planar bottom surface of the floor plate (220) of the lower chamber (200).

14. Use of a heating system according to claim 13 for separating motile cells from other components of a cell sample, wherein, preferably, the heating system is used in a method according to any of claims 12;
wherein, further preferably, during incubation of the device (100) in step 6), the temperature of the heating block is set to a lower temperature than the temperature of the heated lid,
wherein, more preferably, the temperature of the heating block is set to a temperature in a range of about 30°C to about 37°C, even more preferably in a range of about 32°C to about 36°C, most preferably to about 35°C, and/or the temperature of the heated lid is set to a temperature in a range of about 35°C to about 40°C, even more preferably in a range of about 36°C to about 38°C, most preferably to about 37°C.

15. A kit for the separation of motile cells from other components of a cell sample, the kit comprising
a) a device (100) according to any of claims 1-9; and
one or more of, optionally all of:
b) one or more syringes for inserting a cell sample into or extracting a cell sample from the lower chamber (200);
c) one or more syringes for inserting conditioning medium into and/or extracting conditioning medium from the upper chamber (300);
d) a sample buffer;
e) a conditioning medium;
f) a sterile collection container for the cell sample;
g) one or more sterile capillaries for aspirating and/or dispensing the cell sample and/or the conditioning medium into the corresponding syringes of b) and/or c); or
h) a heating system according to claim 13.
